# EUROPÄISCHE PATENTSCHRIFT

(11) **EP 1 208 077 B1**
(45) Veröffentlichungstag und Bekanntmachung des Hinweises auf die Patenterteilung: **21.04.2004**
(21) Anmeldenummer: 01936066.8
(22) Anmeldetag: 10.03.2001
(51) Int. Cl.: C07C 215/76, C07C 215/78, C07C 215/80, C07C 217/80, C07C 255/59, C07C 225/22, C07C 323/31, C07D 317/58, C07D 209/08, A61K 7/13, D06P 1/32

(54) **2-HYDROXY-5-AMINO-BIPHENYL-DERIVATE SOWIE DIESE VERBINDUNGEN ENTHALTENDE OXIDATIONSHAARFÄRBEMITTEL**
2-HYDROXY-5-AMINO-BIPHENYL-DERIVATIVES AND OXIDATIVE HAIR COLOURING AGENTS CONTAINING SAID COMPOUNDS
DERIVES DE 2-HYDROXY-5-AMINO-BIPHENYLE, AINSI QUE PRODUITS COLORANTS PAR OXYDATION POUR LES CHEVEUX CONTENANT CES COMPOSES

(30) Priorität: 01.07.2000 DE 10032134
(43) Veröffentlichungstag der Anmeldung: 29.05.2002
(73) Patentinhaber: Wella Aktiengesellschaft, 64274 Darmstadt (DE)
(72) Erfinder: CHASSOT, Laurent, CH-1724 Praroman (CH); BRAUN, Hans-Jürgen, CH-3182 Überstorf (CH)
(86) Internationale Anmeldenummer: PCT/EP2001/002704
(87) Internationale Veröffentlichungsnummer: WO 2002/002507

(56) Entgegenhaltungen:
- EP-A- 0 027 679
- DE-A- 2 518 393
- DE-A- 2 659 056
- US-A- 3 901 918
- R.C. WEAST: "CRC handbook of chemistry and physics" 1988 , CRC PRESS , US XP002176454 157200 edition 69 Seite C153, Zeile 3388
- DATABASE CHEMICAL ABSTRACTS [Online] STN; access number 113: 23 572, XP002176455 & A. AVDEENKO: "Structure of products in reaction of N-arenesulfonyl-p-quinonimines with naphthols" ZH. ORG. KHIM., Bd. 25, Nr. 11, 1989, Seiten 2375-2381, ussr
- DATABASE CHEMICAL ABSTRACTS [Online] STN; access number 77: 61436, XP002176456 & E. TITOV ET AL.: "Production of 2-(2-hydroxy-1-naphtyl)-4-aminophenols" KHIM. TEKHNOL., Nr. 22, 1971, Seiten 13-15, Kharkov

## Beschreibung

Die vorliegende Erfindung betrifft unsymmetrische 2-Hydroxy-5-aminobiphenyl-Derivate sowie Mittel zur oxidativen Färbung von Keratinfasem, insbesondere menschlichen Haaren auf der Basis einer Entwicklersubstanz/Kupplersubstanz-Kombination, welche als Entwicklersubstanz 2-Hydroxy-5-amino-biphenyl-Derivate enthalten.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere p-Phenylendiamine und p-Aminophenol eingesetzt, während als Kupplersubstanzen beispielsweise Resorcine, 1-Naphthol, 3-Aminophenole und m-Phenylendiamine zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

Die Verwendung von bestimmten symmetrischen Tetraamino-dihydroxydiphenylen in Oxidationshaarfärbemitteln ist aus der DE-A 26 59 056 bekannt. Die Verwendung von bestimmten symmetrischen Diaminodihydroxy-diphenylen in Oxidationshaarfärbemitteln ist aus der DE-A 25 18 393 bekannt. In der US-A 3 901 918 wird ein Verfahren zur Herstellung von Fluoranverbindungen beschrieben, bei dem als Ausgangsverbindung 2-Hydroxy-5-amino-diphenyl-Hydrochlorid verwendet wird. Das 2-Hydroxy-5-amino-diphenyl ist ebenfalls aus dem CRC Handbook of Chemistry and Physics, Ed. 69 (1988), Seite C-153, bekannt. Aus der EP-A 0 027 679 ist die Verwendung bestimmter Diphenylderivate in pharmazeutischen Präparaten bekannt.

Es wurde nunmehr gefunden, dass blonde bis rote Farbnuancen erhalten werden, wenn man bestimmte 2-Hydroxy-5-amino-biphenyl-Derivate zusammen mit üblichen Kupplerverbindungen zur Kupplung bringt.

Gegenstand der vorliegende Erfindung ist daher ein Mittel zur oxidativen Färbung von Keratinfasem, wie zum Beispiel Wolle, Pelzen, Federn oder Haaren, insbesondere menschlichen Haaren, auf der Basis einer Entwicklersubstanz-Kupplersubstanz-Kombination, welches als Entwicklersubstanz mindestens ein 2-Hydroxy-5-amino-biphenyl-Derivat, welches ausgewählt ist aus der Gruppe bestehend aus 2-Hydroxy-3-chlor-5-amino-biphenyl; 2-Hydroxy-3-methyl-5-amino-biphenyl; 2-Hydroxy-4-chlor-5-amino-biphenyl; 2-Hydroxy-4-methyl-5-amino-biphenyl; 2,2'-Dihydroxy-5-amino-biphenyl; 2,3'-Dihydroxy-5-amino-biphenyl; 2,4'-Dihydroxy-5-amino-biphenyl; 2,5'-Dihydroxy-5-amino-biphenyl; 2,6'-Dihydroxy-5-amino-biphenyl; 2-Hydroxy-5,2'-diamino-biphenyl; 2-Hydroxy-5,3'-diamino-biphenyl; 2-Hydroxy-5,4'-diamino-biphenyl; 2-Hydroxy-5,5'-diamino-biphenyl; 2-Hydroxy-5,6'-diamino-biphenyl; 2,2',3-Trihydroxy-5-amino-biphenyl; 2,2',4'-Trihydroxy-5-amino-biphenyl; 2,2',5'-Trihydroxy-5-amino-biphenyl; 2,2,6'-Trihydroxy-5-amino-biphenyl; 2,3',4'-Trihydroxy-5-amino-biphenyl; 2,3',5'-Trihydroxy-5-amino-biphenyl; 2-Hydroxy-5,2',3'-triamino-biphenyl; 2-Hydroxy-5,2',4-triamino-biphenyl; 2-Hydroxy-5,2',5'-triamino-biphenyl; 2-Hydroxy-5,2',6'-triamino-biphenyl; 2-Hydroxy-5,3',4'-triamino-biphenyl; 2-Hydroxy-5,3',5'-triamino-biphenyl; 2-Hydroxy-5,3',4-triamino-biphenyl; 2-Hydroxy-5,3',5'-triamino-biphenyl; 2-Hydroxy-5-amino-2'-aminomethyl-biphenyl; 2-Hydroxy-5-amino-2'-chlor-biphenyl; 2-Hydroxy-5-amino-2'-cyan-biphenyl; 2-Hydroxy-5-amino-2'-fluor-biphenyl; 2-Hydroxy-5-amino-2'-methoxy-biphenyl; 2-Hydroxy-5-amino-2'-methyl-biphenyl; 2-Hydroxy-5-amino-2'-methylsulfanyl-biphenyl; 2-Hydroxy-5-amino-2'-nitro-biphenyl; 2-Hydroxy-5-amino-3'-aminomethyl-biphenyl; 2-Hydroxy-5-amino-3'-chlor-biphenyl; 2-Hydroxy-5-amino-3'-cyan-biphenyl; 2-Hydroxy-5-amino-3-fluor-biphenyl; 2-Hydroxy-5-amino-3'-methoxy-biphenyl; 2-Hydroxy-5-amino-3'-methyl-biphenyl; 2-Hydroxy-5-amino-3'-methylsulfanyl-biphenyl; 2-Hydroxy-5-amino-3'-nitro-biphenyl; 2-Hydroxy-5-amino-4'-aminomethyl-biphenyl; 2-Hydroxy-5-amino-4'-chlor-biphenyl; 2-Hydroxy-5-amino-4'-cyan-biphenyl; 2-Hydroxy-5-amino-4'-fluor-biphenyl; 2-Hydroxy-5-amino-4'-methoxy-biphenyl; 2-Hydroxy-5-amino-4'-methyl-biphenyl; 2-Hydroxy-5-amino-4'-methylsulfanyl-biphenyl; 2-Hydroxy-5-amino-4'-nitro-biphenyl; 2-Hydroxy-5-amino-5'-aminomethyl-biphenyl; 2-Hydroxy-5-amino-5'-chlor-biphenyl; 2-Hydroxy-5-amino-5'-cyan-biphenyl; 2-Hydroxy-5-amino-5'-fluor-biphenyl; 2-Hydroxy-5-amino-5'-methoxy-biphenyl; 2-Hydroxy-5-arnino-5'-methyl-biphenyl; 2-Hydroxy-5-amino-5'-methylsulfanyl-biphenyl; 2-Hydroxy-5-amino-5'-nitro-biphenyl; 2-Hydroxy-5-amino-6'-aminomethyl-biphenyl; 2-Hydroxy-5-amino-6'-chlor-biphenyl; 2-Hydroxy-5-amino-6'-cyan-biphenyl; 2-Hydroxy-5-amino-6'-fluor-biphenyl; 2-Hydroxy-5-amino-6'-methoxy-biphenyl; 2-Hydroxy-5-amino-6'-methyl-biphenyl; 2-Hydroxy-5-amino-6'-methylsulfanyl-biphenyl; 2-Hydroxy-5-amino-6'-nitro-biphenyl; 2,2'-Dihydroxy-5-amino-3'-methyl-biphenyl, 2,2'-Dihydroxy-5-amino-4'-methyl-biphenyl; 2,2'-Dihydroxy-5-amino-5'-methyl-biphenyl; 2,2'-Dihydroxy-5-amino-6'-methyl-biphenyl; 2,3-Dihydroxy-5-amino-4'-methyl-biphenyl; 2,3'-Dihydroxy-5-amino-5'-methyl-biphenyl; 2-Hydroxy-5-amino-2',3'-dimethoxy-biphenyl; 2-Hydroxy-5-amino-2',3-dirnethylbiphenyl; 2-Hydroxy-5-amino-2',4'-dimethoxy-biphenyl; 2-Hydroxy-5-amino-2',4'-dimethyl-biphenyl; 2-Hydroxy-5-amino-2',5'-dimethoxy-biphenyl; 2-Hydroxy-5-amino-2',5'-dimethyl-biphenyl; 2-Hydroxy-5-amino-2',6'-dimethoxy-biphenyl; 2-Hydroxy-5-amino-2',6'-dimethyl-biphenyl; 2-Hydroxy-5-amino-3',4'-dimethoxy-biphenyl; 2-Hydroxy-5-amino-3',4'-dimethyl-biphenyl; 2-Hydroxy-5-amino-3',5'-dimethoxy-biphenyl; 2-Hydroxy-5-amino-3',5'-dimethyl-biphenyl; 2-Hydroxy-5-amino-2'-methoxy-3'-methyl-biphenyl; 2-Hydroxy-5-amino-2'-methoxy-4'-methyl-biphenyl; 2-Hydroxy-5-amino-2'-methoxy 5'-methyl-biphenyl; 2-Hydroxy-5-amino-2'-methoxy-6'-methyl-biphenyl; 2-Hydroxy-5-amino-3'-methoxy-4'-methyl-biphenyl; 2-Hydroxy-5-amino-3'-methoxy-5'-methyl-biphenyl; 4-Amino-2-benzo[1,3]dioxol-5-yl-phenol; 4-Amino-2-benzo[2,4]dioxol-5-yl-phenol und 2-Hydroxy-5-amino-4'-(2-hydroxy-ethoxy)-biphenyl, oder deren physiologisch verträglichen, wasserlöslichen Salze, enthält.

Besonders hervorragend geeignete 2-Hydroxy-5-amino-biphenyl-Derivate im Sinne der Gesamterfindung sind 2-Hydroxy-5-amino-biphenyl, 2,4'-Dihydroxy-5-amino-biphenyl, 2-Hydroxy-5-amino-4'-(2"-hydroxyethoxy)-biphenyl, 2,4'-Dihydroxy-5-amino-2'-methyl-biphenyl, 2-Hydroxy-5-amino-4'-(2"-hydroxyethyl)-biphenyl, 2-Hydroxy-5,4'-diamino-biphenyl oder deren physiologisch verträgliche Salze.

Die 2-Hydroxy-5-amino-biphenyl-Derivate können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Das 2-Hydroxy-5-amino-biphenyl-Derivat ist in dem erfindungsgemäßen Färbemittel in einer Menge von etwa 0,005 bis 20 Gewichtsprozent enthalten, wobei eine Menge von etwa 0,01 bis 5,0 Gewichtsprozent und insbesondere 0,1 bis 2,5 Gewichtsprozent bevorzugt ist.

Als Kupplersubstanzen kommen vorzugsweise 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethyl-amino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzo-dioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion in Betracht.

Obwohl die vorteilhaften Eigenschaften der hier beschriebenen 2-Hydroxy-5-amino-biphenyl-Derivate es nahelegen, diese als alleinige Entwicklersubstanz zu verwenden, ist es selbstverständlich auch möglich, die 2-Hydroxy-5-amino-biphenyl-Derivate gemeinsam mit bekannten Entwicklersubstanzen, wie zum Beispiel 1,4-Diaminobenzol, 2,5-Diaminotoluol, 2,5-Diaminophenylethylalkohol, 4-Aminophenol und seinen Derivaten, beispielsweise 4-Amino-3-methyl-phenol, 4,5-Diaminopyrazol-Derivaten, beispielsweise 4,5-Diamino-1-(2-hydroxyethyl)-pyrazol, oder Tetraaminopyrimidinen, einzusetzen. Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6,0 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Uberschuß oder Unterschuß (beispielsweise in einem Verhältnis (Kuppler : Entwickler) von 1:2 bis 1:0,5) vorhanden sind.

Weiterhin kann das erfindungsgemäße Färbemittel zusätzlich weitere Farbkomponenten, beispielsweise 6-Amino-2-methylphenol und 2-Amino-5-methylphenol, sowie ferner übliche direktziehende Farbstoffe aus der Gruppe der sauren oder basischen Farbstoffe, Triphenylmethanfarbstoffe, Anthrachinonfarbstoffe, aromatischen Nitrofarbstoffe, Azofarbstoffe, Lebensmittelfarbstoffe oder Dispersionsfarbstoffe, enthalten. Die Färbemittel können diese Farbkomponenten in einer Menge von etwa 0,1 bis 4,0 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, UV-Absorber, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen hingegen anorganische oder organische Säuren, wie zum Beispiel Phosphorsäure, Essigsäure, Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3- bis 12prozentigen, vorzugsweise 6prozentigen, wässrigen Lösung, aber auch Luftsauerstoff in Betracht. Wird eine 6prozentige WasserstoffperoxidLösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Färbemittel mit einem Gehalt an 2-Hydroxy-5-amino-biphenyl-Derivaten ermöglichen Färbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Färbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften der Färbemittel gemäß der vorliegenden Anmeldung zeigen sich weiterhin darin, daß diese Mittel insbesondere auch eine Anfärbung von ergrauten, chemisch nicht vorgeschädigten Haaren problemlos und mit guter Deckkraft ermöglichen. Die in dem erfindungsgemäßen Mittel verwendeten 2-Hydroxy-5-aminobiphenyl-Derivate der Formel (I) sind in Wasser löslich und ermöglichen Färbungen mit hoher Farbintensität und ausgezeichneter Farbechtheit, insbesondere was die Lichtechtheit, Waschechtheit und Reibeechtheit anbetrifft. Sie weisen weiterhin eine ausgezeichnete Lagerstabilität, insbesondere als Bestandteil der vorstehend beschriebenen Färbemittel, auf.

Ein weiterer Gegenstand der vorliegenden Erfindung sind neue 2-Hydroxy-5-amino-biphenyl-Derivate, welche ausgewählt sind aus 2-Hydroxy-3-chlor-5-amino-biphenyl; 2-Hydroxy-3-methyl-5-amino-biphenyl; 2-Hydroxy-4-chlor-5-amino-biphenyl; 2-Hydroxy-4-methyl-5-amino-biphenyl; 2,2-Dihydroxy-5-amino-biphenyl; 2,3-Dihydroxy-5-amino-biphenyl; 2,4'-Dihydroxy-5-amino-biphenyl; 2,5'-Dihydroxy-5-amino-biphenyl; 2,6'-Dihydroxy-5-amino-biphenyl; 2-Hydroxy-5,2'-diamino-biphenyl; 2-Hydroxy-5,3'-diamino-biphenyl; 2-Hydroxy-5,4'-diamino-biphenyl; 2-Hydroxy-5,5'-diamino-biphenyl; 2-Hydroxy-5,6'-diamino-biphenyl; 2,2',3'-Trihydroxy-5-amino-biphenyl; 2,2',4'-Trihydroxy-5-amino-biphenyl; 2,2',5'-Trihydroxy-5-amino-biphenyl; 2,2',6'-Trihydroxy-5-amino-biphenyl; 2,3',4'-Trihydroxy-5-amino-biphenyl; 2,3',5'-Trihydroxy-5-amino-biphenyl; 2-Hydroxy-5,2',3'-triamino-biphenyl; 2-Hydroxy-5,2',4'-triamino-biphenyl; 2-Hydroxy-5,2,5'-triamino-biphenyl; 2-Hydroxy-5,2',6'-triamino-biphenyl; 2-Hydroxy-5,3',4'-triamino-biphenyl; 2-Hydroxy-5,3',5'-triamino-biphenyl; 2-Hydroxy-5,3',4'-triamino-biphenyl; 2-Hydroxy-5,3',5'-triamino-biphenyl; 2-Hydroxy-5-amino-2'-aminomethyl-biphenyl; 2-Hydroxy-5-amino-2'-chlor-biphenyl; 2-Hydroxy-5-amino-2'-cyan-biphenyl; 2-Hydroxy-5-amino-2'-fluor-biphenyl; 2-Hydroxy-5-amino-2'-methoxy-biphenyl; 2-Hydroxy-5-amino-2'-methyl-biphenyl; 2-Hydroxy-5-amino-2'-methylsulfanyl-biphenyl; 2-Hydroxy-5-amino-2'-nitro-biphenyl; 2-Hydroxy-5-amino-3'-aminomethyl-biphenyl; 2-Hydroxy-5-amino-3'-chlor-biphenyl; 2-Hydroxy-5-amino-3'-cyan-biphenyl; 2-Hydroxy-5-amino-3'-fluor-biphenyl; 2-Hydroxy-5-amino-3'-methoxy-biphenyl; 2-Hydroxy-5-amino-3'-methyl-biphenyl; 2-Hydroxy-5-amino-3'-methylsulfanyl-biphenyl; 2-Hydroxy-5-amino-3'-nitro-biphenyl; 2-Hydroxy-5-amino-4'-aminomethyl-biphenyl; 2-Hydroxy-5-amino-4'-cyan-biphenyl; 2-Hydroxy-5-amino-4'-fluor-biphenyl; 2-Hydroxy-5-amino-4'-methoxy-biphenyl; 2-Hydroxy-5-amino-4'-methyl-biphenyl; 2-Hydroxy-5-amino-4'-methylsulfanyl-biphenyl; 2-Hydroxy-5-amino-4'-nitro-biphenyl; 2-Hydroxy-5-amino-5'-aminomethyl-biphenyl; 2-Hydroxy-5-amino-5'-chlor-biphenyl; 2-Hydroxy-5-amino-5'-cyan-biphenyl; 2-Hydroxy-5-amino-5'-fluor-biphenyl; 2-Hydroxy-5-amino-5'-methoxy-biphenyl; 2-Hydroxy-5-amino-5'-methyl-biphenyl; 2-Hydroxy-5-amino-5'-methylsulfanyl-biphenyl; 2-Hydroxy-5-amino-5-nitro-biphenyl; 2-Hydroxy-5-amino-6'-aminomethyl-biphenyl; 2-Hydroxy-5-amino-6'-chlor-biphenyl; 2-Hydroxy-5-amino-6'-cyan-biphenyl; 2-Hydroxy-5-amino-6'-fluor-biphenyl; 2-Hydroxy-5-amino-6'-methoxy-biphenyl; 2-Hydroxy-5-amino-6'-methyl-biphenyl; 2-Hydroxy-5-amino-6-methylsulfanyl-biphenyl; 2-Hydroxy-5-amino-6'-nitro-biphenyl; 2,2'-Dihydroxy-5-amino-3'-methyl-biphenyl; 2,2'-Dihydroxy-5-amino-4'-methyl-biphenyl; 2,2'-Dihydroxy-5-amino-5'-methyl-biphenyl; 2,2'-Dihydroxy-5-amino-6'-methyl-biphenyl; 2,3'-Dihydroxy-5-amino-4'-methyl-biphenyl; 2,3'-Dihydroxy-5-amino-5'-methyl-biphenyl; 2-Hydroxy-5-amino-2',3'-dimethoxy-biphenyl; 2-Hydroxy-5-amino-2',3'-dimethyl-biphenyl; 2-Hydroxy-5-amino-2',4'-dimethoxy-biphenyl; 2-Hydroxy-5-amino-2',4'-dimethyl-biphenyl; 2-Hydroxy-5-amino-2',5'-dimethoxy-biphenyl; 2-Hydroxy-5-amino-2',5'-dimethyl-biphenyl; 2-Hydroxy-5-amino-2',6'-dimethoxy-biphenyl; 2-Hydroxy-5-amino-2',6'-dimethyl-biphenyl; 2-Hydroxy-5-amino-3,4-dimethoxy-biphenyl, 2-Hydroxy-5-amino-3',4'-dimethyl-biphenyl; 2-Hydroxy-5-amino-3',5'-dimethoxy-biphenyl; 2-Hydroxy-5-amino-3',5'-dimethyl-biphenyl; 2-Hydroxy-5-amino-2'-methoxy-3'-methyl-biphenyl; 2-Hydroxy-5-amino-2'-methoxy-4'-methyl-biphenyl; 2-Hydroxy-5-amino-2'-methoxy-5'-methyl-biphenyl; 2-Hydroxy-5-amino-2'-methoxy-6'-methyl-biphenyl; 2-Hydroxy-5-amino-3'-methoxy-4'-methyl-biphenyl; 2-Hydroxy-5-amino-3'-methoxy-5'-methyl-biphenyl; 4-Amino-2-benzo[1,3]dioxol-5-yl-phenol; 4-Amino-2-benzo[2,4]dioxol-5-yl-phenol und 2-Hydroxy-5-amino-4'-(2-hydroxy-ethoxy)-biphenyl oder deren physiologisch verträglichen, wasserlöslichen Salzen.

Die Herstellung der erfindungsgemäßen 2-Hydroxy-5-amino-biphenyl-Derivate kann unter Verwendung von bekannten Syntheseverfahren, beispielsweise in Analogie zu dem in den nachfolgenden Herstellungsbeispielen beschriebenen allgemeinen Verfahren, erfolgen.

Die nachfolgenden Beispiele sollen den Gegenstand der Erfindung näher erläutern, ohne ihn darauf zu beschränken.

### Beispiele

### Beispiele 1: Synthese von 2-Hydroxy-5-amino-biphenyl Hydrochlorid

### A. Synthese von 2-Brom-4-nitro-phenol

Der 2-Brom-4-nitro-phenol wird durch Umsetzung von 4-Nitro-phenol mit N-Bromsuccinimid dargestellt. Die experimentelle Vorschrift dieser Herstellungsmethode wird von T. Oberhouser in J. Org. Chem. 1997 (62), Seite 4504 beschrieben.

### B. Synthese von 2-Brom-1-methoxymethoxy-4-nitro-benzol

Zu einer Lösung von 15,3 g (70,0 mmol) 2-Brom-4-nitro-phenol aus Stufe **A** in 250 ml Tetrahydrofuran (THF) werden bei 0°C portionsweise insgesamt 4,2 g (140 mmol) einer Natriumhydrid-Dispersion (55% in Öl) gegeben. Das Reaktionsgemisch wird anschliessend 50 Minuten lang bei 0°C gerührt und sodann mit 1,83 g (19,4 mmol) Chlormethylmethylether versetzt. Das Reaktionsgemisch wird nochmals für 1 Stunde bei 0°C gerührt und anschliessend aufgearbeitet. Hierzu wird das Reaktionsgemisch auf Eis gegossen, mit Essigsäureethylester extrahiert und die organische Phase mit einer gesättigten wässerigen Kochsalz-Lösung gewaschen, über Natriumsulfat getrocknet und nach Filtration eingeengt. Der Rückstand wird an Kieselgel mit Petrolether/Essigsäureethylester (9:1) gereinigt.
Es werden 15,8 g (80% der Theorie) 2-Brom-1-methoxymethoxy-4-nitrobenzol erhalten.
¹H-NMR (300 MHz, CDCl₃): δ = 8,48 (s, 1H), 7,08 (d, 1H), 8,16 (d; 1H), 7,26 (d, 1H), 5,36 (s, 2H), 3,53 (s, 3H)

### C. Synthese von 2-Hydroxy-5-nitro-biphenyl

5,3 g (0,02 mol) 2-Brom-1-methoxymethoxy-4-nitro-benzol aus Stufe **B** und 2,80 g (0,023 mol) Phenylborsäure werden unter Argon in 70 ml 1,2-Dimethoxyethan gelöst. Anschließend werden 0,5 g (0,0005 mol) Tetrakis-(triphenylphosphin)-palladium und 13 ml einer 2normalen Kaliumcarbonatlösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in einer Mischung von 40 ml Ethanol und 15 ml einer 2,9molaren ethanolische Salzsäurelösung auf 50 °C erwärmt. Nach Neutralisation mit NaOH wird das Lösungsmittel am Rotationsverdampfer abdestilliert und der Rückstand durch Vakuumdestillation gereinigt.
Es werden 3,5 g (82 % der Theorie) 2-Hydroxy-5-nitro-biphenyl erhalten.
¹H-NMR (300 MHz, CDCl₃): δ= 8,2 (m, 2H), 7,55 (m, 2H), 7,49 (m, 3H), 7,08 (d; 1H), 6,14 (s, 1H)

### D. Synthese von 2-Hydroxy-5-amino-biphenyl Hydrochlorid

2,9 g (13,5 mmol) 2-Hydroxy-5-nitro-biphenyl aus Stufe **C** werden in 40 ml Ethanol gelöst und unter Zusatz von 600 mg eines Palladium-Aktivkohle-Katalysators (10%ig) bei 25 °C hydriert. Nach Aufnahme der theoretisch erforderlichen Wasserstoffmenge wird vom Katalysator abfiltriert. Nach dem Einengen der Lösung am Rotationsverdampfer wird das Phenol durch Vakuumdistillation gereinigt und mit 2,9molaren ethanolische Salzsäurelösung versetzt.
Daus ausgefallene Produkt wird abfiltriert und getrocknet.
Es werden 0,5 g (17 % der Theorie) 2-Hydroxy-5-amino-biphenyl mit einem Schmelzpunkt von 130-132°C erhalten.

| CHN-Analyse:(C₁₂H₁₂NOCl) | | | |
|---|---|---|---|
| | % C | % H | % N |
| berechnet | 65,02 | 5,46 | 6,32 |
| gefunden | 62,28 | 5,45 | 6,05 |

### Beispiele 2: Synthese von substituierten 2-Hydroxy-5-amino-biphenyl-Derivaten der allgemeinen Formel (I) (Allgemeine Synthesevorschrift)

### A. Synthese von N-(3-Brom-4-hydroxy-phenyl)-carbaminsäure-tert.butylester

Zu einer Suspension von 10 g (47,8 mmol) N-(4-Hydroxy-phenyl)-carbaminsäure-tert.butylester in 100 ml Chloroform tropft man bei 0 °C innerhalb von 2 Stunden eine Lösung von 9,4 g (52,8 mmol) N-Brom-succinimid in 450 ml Chloroform. Die Reaktionsmischung wird füe weitere 15 Minuten weitergerührt, zweimal mit Wasser (zunächst 400 ml, dann 200 ml) gewaschen, über Magnesiumsulfat getrocknet, filtriert und partiell eingeengt. Der Rückstand wird unter Rühren mit Hexan versetzt, wobei sich ein Niederschlag bildet. Der Niederschlag wird abfiltriert und mit Hexan gewaschen.
Es werden 9,7 g (70 % der Theorie) N-(3-Brom-4-hydroxy-phenyl)-carbaminsäure-tert.butylester erhalten.
¹H-NMR (300 MHz, CDCl₃): δ = 7,68 (br s, 1H), 7,05 (dd, 1H), 6,93 (d, 1H), 6,37 (br s; 2H), 5,39 (s, 1H), 1,51 (s, 9H)

### B. Synthese von N-(3-Brom-4-ethoxymethoxy-phenyl)-carbaminsäure-tert.butylester

Zu einer Lösung von 5,0 g (17,4 mmol) N-(3-Brom-4-hydroxy-phenyl)-carbaminsäure-tert.butylester aus Stufe **A** in 60 ml THF gibt man bei 0°C portionsweise 0,76 g (17,4 mmol) einer Natriumhydrid-Dispersion (55% in Öl). Das Gemisch wird anschließend 50 Minuten lang bei 0°C gerührt. Dann gibt man 1,83 g (19,4 mmol) Chlormethyl-ethylether hinzu und rührt das Gemisch 1 Stunde lang bei 0 °C. Anschliessend wird as Reaktionsgemisch auf Eis gegossen, mit Essigsäureethylester extrahiert und die organische Phase mit einer gesättigten wässerigen Kochsalz-Lösung gewaschen, über Na2SO4 getrocknet und nach Filtration eingeengt. Der Rückstand wird an Kieselgel mit Petrolether/Essigsäureethylester (9:1) gereinigt.
Es werden 4,8 g (80% der Theorie) N-(3-Brom-4-hydroxy-phenyl)-carbaminsäure-tert.butylester erhalten.
¹H-NMR (300 MHz, CDCl₃): δ= 7,66 (d, 1H), 7,16 (dd, 1H), 7,08 (d, 1H), 5,23 (s; 2H), 3,77 (q, 2H), 1,51 (s, 9H), 1,22 (t, 3H)

### C. Synthese von N-[4-Ethoxymethoxy-3-(4,4,5,5-tetramethyl-[1,3,2]dioxaborolan-2-yl)-phenyl]-carbaminsäure-tert.butylester

Eine Mischung von 7,0 g (20.2 mmol) N-(3-Brom-4-ethoxymethoxyphenyl)-carbaminsäure-tert.butylester aus Stufe B, 12,8 g (50.6 mmol) Diboron-pinacolester, 2,0 g (2,9 mmol) Dichloro (1,1'-bis(diphenyl-phosphino)ferrocene)palladium (PdCl₂(dppf)) und 6,2 g (63,2 mmol) Kaliumacetat werden unter Argon mit 210 ml entgastem Dioxan versetzt. Das Gemisch wird 26 Stunden lang bei 80 °C gerührt und anschliessend mit einem Gemisch aus 4,2 g (16,9 mmol) Diboronpinacolester und 700 mg (0,95 mmol) PdCl₂(dppf) versetzt. Das Reaktionsgemisch wird weitere 14 Stunden lang bei 80 °C gerührt und sodann auf Wasser gegossen, mit Essigsäureethylester extrahiert. Die organische Phase wird anschliessend mit einer gesättigten wässerigen Kochsalz-Lösung gewaschen, über Natriumsulfat getrocknet und nach Filtration eingeengt. Das erhaltene Rohprodukt wird an desaktiviertem Kieselgel* mit Hexan/Essigsäureethylester (1:1) gereinigt.
Es werden 5,30 g (61 % der Theorie) N-[4-Ethoxymethoxy-3-(4,4,5,5-tetramethyl-[1,3,2]dioxaborolan-2-yl)-phenyl]- carbaminsäure-tert.butylester erhalten.

### D. Synthese von substituierten 2-Hydroxy-5-amino-biphenyl-Derivaten

0,036 g (0,0001 mol) N-[4-Ethoxymethoxy-3-(4,4,5,5-tetramethyl-[1,3,2]dioxaborolan-2-yl)-phenyl]- carbaminsäure-tert.butylester aus Stufe **C** und 0,013 mol des entsprechenden Bromderivates werden unter Argon in 70 ml 1,2-Dimethoxyethan gelöst. Anschließend werden 0,5 g (0,0005 mol) Tetrakis-(triphenylphosphin)-palladium und 13 ml einer 2normalen Kaliumcarbonatlösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann über Magnesiumsulfat getrocknet. Das Lösungsmittel wird am Rotationsverdampfer im Vakuum abdestilliert und der Rückstand an Kieselgel mit Petrolether/Essigsäureethylester (9:1) gereinigt. Das so erhaltene Produkt wird in 40 ml Ethanol auf 50 °C erwärmt. Anschliessend werden zur Herstellung des Hydrochlorides 15 ml einer 2,9 molaren ethanolische Salzsäurelösung zugetropft. Der Niederschlag wird abfiltriert, zweimal mit 10 ml Ethanol gewaschen und sodann getrocknet.

### 2.1. 2-Hydroxy-5-amino-2'-methyl-biphenyl Hydrochlorid

Verwendetes Bromderivat: 2-Brom-toluol
Ausbeute: 95 % der Theorie
Masspektrum: MH⁺ 199 (100)

### 2.2. 2-Hydroxy-5-amino-3'-methyl-biphenyl Hydrochlorid

Verwendetes Bromderivat: 3-Brom-toluol
Ausbeute: 84 % der Theorie
Masspektrum: MH⁺ 199 (100)

### 2.3. 2-Hydroxy-5-amino-4'-methyl-biphenyl Hydrochlorid

Verwendetes Bromderivat: 4-Brom-toluol
Ausbeute: 88 % der Theorie
Masspektrum: MH⁺ 199 (100)

### 2.4. 2-Hydroxy-5,4'-diamino-biphenyl Hydrochlorid

Verwendetes Bromderivat: 4-Brom-anilin
Ausbeute: 91 % der Theorie
Masspektrum: MH⁺ 237 (100)

### 2.5. 5-Amino-biphenyl-2,4'-diol Hydrochlorid

Verwendetes Bromderivat: 4-Brom-phenol
Ausbeute: 96 % der Theorie
Masspektrum: MH⁺ 201 (100)

### 2.6. 5-Amino-biphenyl-2,3'-diol Hydrochlorid

Verwendetes Bromderivat: 3-Brom-phenol
Ausbeute: 95 % der Theorie
Masspektrum: MH⁺ 201 (100)

### 2.7. 2-Hydroxy-5-amino-4-fluor-biphenyl Hydrochlorid

Verwendetes Bromderivat: 1-Brom-4-Fluor-benzol
Ausbeute: 95 % der Theorie
Masspektrum: MH⁺ 203 (100)

### 2.8. 2-Hydroxy-5-amino-2'-fluor-biphenyl Hydrochlorid

Verwendetes Bromderivat: 1-Brom-2-Fluor-benzol
Ausbeute: 95 % der Theorie
Masspektrum: MH⁺ 203 (100)

### 2.9. 5'-Amino-2'-hydroxy-biphenyl-4-carbonitril Hydrochlorid

Verwendetes Bromderivat: 4-Brom-benzonitril
Ausbeute: 95 % der Theorie
Masspektrum: MH⁺ 210 (100)

### 2.10. 5'-Amino-2'-hydroxy-biphenyl-3-carbonitril Hydrochlorid

Verwendetes Bromderivat: 3-Brom-benzonitril
Ausbeute: 95 % der Theorie
Masspektrum: MH⁺ 210 (100)

### 2.11. 2-Hydroxy-5-amino-2'-ethyl-biphenyl Hydrochlorid

Verwendetes Bromderivat: 1-Brom-2-ethyl-benzol
Ausbeute: 95 % der Theorie
Masspektrum: MH⁺ 213 (100)

### 2.12. 2-Hydroxy-5-amino-4'-ethyl-biphenyl Hydrochlorid

Verwendetes Bromderivat: 1-Brom-4-ethyl-benzol
Ausbeute: 80 % der Theorie
Masspektrum: MH⁺ 213 (100)

### 2.13. 2-Hydroxy-5-amino-2',4'-dimethyl-biphenyl Hydrochlorid

Verwendetes Bromderivat: 6-Brom-m-xylol
Ausbeute: 95 % der Theorie
Masspektrum: MH⁺ 213 (100)

### 2.14. 2-Hydroxy-5-amino-2',3'-dimethyl-biphenyl Hydrochlorid

Verwendetes Bromderivat: 6-Brom-o-xylol
Ausbeute: 95 % der Theorie
Masspektrum: MH⁺ 213 (100)

### 2.15. 2-Hydroxy-5-amino-2',5'-dimethyl-biphenyl Hydrochlorid

Verwendetes Bromderivat: 2-Brom-p-xylol
Ausbeute: 95 % der Theorie
Masspektrum: MH⁺ 213 (100)

### 2.16. 2-Hydroxy-5-amino-3',5'-dimethyl-biphenyl Hydrochlorid

Verwendetes Bromderivat: 5-Brom-m-xylol
Ausbeute: 95 % der Theorie
Masspektrum: MH⁺ 213 (100)

### 2.17. 2-Hydroxy-5-amino-3'-aminomethyl-biphenyl Hydrochlorid

Verwendetes Bromderivat: 3-Brom-benzylamin
Ausbeute: 87 % der Theorie
Masspektrum: MH⁺ 251 (100)

### 2.18. 2-Hydroxy-5-amino-4'-methoxy-biphenyl Hydrochlorid

Verwendetes Bromderivat: 4-Brom-anisol
Ausbeute: 99 % der Theorie
Masspektrum: MH⁺ 215 (100)

### 2.19. 2-Hydroxy-5-amino-2'-methoxy-biphenyl Hydrochlorid

Verwendetes Bromderivat: 2-Brom-anisol
Ausbeute: 79 % der Theorie
Masspektrum: MH⁺ 215 (100)

### 2.20. 2-Hydroxy-5-amino-3'-methoxy-biphenyl Hydrochlorid

Verwendetes Bromderivat: 3-Brom-anisol
Ausbeute: 97 % der Theorie
Masspektrum: MH⁺ 215 (100)

### 2.21. 2-Hydroxy-5-amino-5'-fluor-2'-methyl-biphenyl Hydrochlorid

Verwendetes Bromderivat: 2-Brom-4-fluor-toluol
Ausbeute: 82 % der Theorie
Masspektrum: MH⁺ 217 (100)

### 2.22. 4-Amino-2-(1H-indol-5-yl)-phenol Hydrochlorid

Verwendetes Bromderivat: 5-Brom-indol
Ausbeute: 95 % der Theorie
Masspektrum: MH⁺ 224 (100)

### 2.23. 1-(5'-Amino-2'-hydroxy-biphenyl-3-yl)-ethanone Hydrochlorid

Verwendetes Bromderivat: 2-Brom-acetophenon
Ausbeute: 56 % der Theorie
Masspektrum: MH⁺ 227 (100)

### 2.24. 4-Amino-2-benzo[1,3]dioxol-5-yl-phenol Hydrochlorid

Verwendetes Bromderivat: 4-Brom-1,2-methylendioxy-benzol
Ausbeute: 93 % der Theorie
Masspektrum: MH⁺ 229 (100)

### 2.25. 2-Hydroxy-5-amino-3'-ethoxy-biphenyl Hydrochlorid

Verwendetes Bromderivat: 1-Brom-3-Ethoxy-benzol
Ausbeute: 78 % der Theorie
Masspektrum: MH⁺ 229 (100)

### 2.26. 2-Hydroxy-5-amino-4'-methoxy-2'-methyl-biphenyl Hydrochlorid

Verwendetes Bromderivat: 2-Brom-5-methoxy-toluol
Ausbeute: 93 % der Theorie
Masspektrum: MH⁺ 229 (100)

### 2.27. 2-Hydroxy-5-amino-2'-(2-hydroxy-ethyl)-biphenyl Hydrochlorid

Verwendetes Bromderivat: 2-(2-Bromphenyl)-ethanol
Ausbeute: 78 % der Theorie
Masspektrum: MH⁺ 229 (100)

### 2.28. 2-Hydroxy-5-amino-4'-nitro-biphenyl Hydrochlorid

Verwendetes Bromderivat: 4-Brom-nitro-benzol
Ausbeute: 93 % der Theorie
Masspektrum: MH⁺ 230 (100)

### 2.29. 2-Hydroxy-5-amino-4'-methylsulfanyl-biphenyl Hydrochlorid

Verwendetes Bromderivat: 1-Brom-4-methylmercapto-benzol
Ausbeute: 93 % der Theorie
Masspektrum: MH⁺ 231 (100)

### 2.30. 2-Hydroxy-5-amino-4'-tert-butyl-biphenyl Hydrochlorid

Verwendetes Bromderivat: 1-Brom-4-tert.-butyl-benzol
Ausbeute: 89 % der Theorie
Masspektrum: MH⁺ 241 (100)

### 2.31. 2-Hydroxy-5-amino-2',4'-dimethoxy-biphenyl Hydrochlorid

Verwendetes Bromderivat: 6-Brom-1,3-dimethoxy-benzol
Ausbeute: 88 % der Theorie
Masspektrum: MH⁺ 245 (100)

### 2.32. 2-Hydroxy-5-amino-2',5'-dimethoxy-biphenyl Hydrochlorid

Verwendetes Bromderivat: 2-Brom-2,5-dimethoxy-benzol
Ausbeute: 88 % der Theorie
Masspektrum: MH⁺ 245 (100)

### 2.33. 2-Hydroxy-5-amino-4'-(2-hydroxy-ethoxyl-biphenyl Hydrochlorid

Verwendetes Bromderivat: 1-Brom-4-(2-hydroxy-ethoxy)-benzol
Ausbeute: 88 % der Theorie
Masspektrum: MH⁺ 245 (100)

### 2.34. 2-Hydroxy-5-amino-4'-trifluormethyl-biphenyl Hydrochlorid

Verwendetes Bromderivat: 1-Brom-4-trifluormethyl-benzol
Ausbeute: 86 % der Theorie
Masspektrum: MH⁺ 253 (100)

### 2.35. 2-Hydroxy-5-amino-3',4'-dimethyl-biphenyl Hydrochlorid

Verwendetes Bromderivat: 4-Brom-o-xylol
Ausbeute: 95 % der Theorie
Masspektrum: MH⁺ 213 (100)

### 2.36. 2-Hydroxy-5-amino-4'-ethoxy-biphenyl Hydrochlorid

Verwendetes Bromderivat: 1-Brom-4-ethoxy-benzol
Ausbeute: 93 % der Theorie
Masspektrum: MH⁺ 229 (100)

### 2.37. 2-Hydroxy-5-amino-2'-methylsulfanyl-biphenyl Hydrochlorid

Verwendetes Bromderivat: 1-Brom-2-Methylmercapto-benzol
Ausbeute: 93 % der Theorie
Masspektrum: MH⁺ 231 (100)

### 2.38. 2-Hydroxy-5-amino-3'-fluor-biphenyl Hydrochlorid

Verwendetes Bromderivat: 1-Brom-3-Fluor-benzol
Ausbeute: 95 % der Theorie
Masspektrum: MH⁺ 203 (100)

### 2.39. 5-Amino-2'-methyl-biphenyl-2,4'-diol Hydrochlorid

Verwendetes Bromderivat: 4-Brom-3-methyl-phenol
Ausbeute: 94 % der Theorie
Masspektrum: MH⁺ 215 (100)

### 2.40. 2-Hydroxy-5-amino-4'-(2-hydroxy-ethyl)-biphenyl Hydrochlorid

Verwendetes Bromderivat: 1-Brom-4-(2-Hydroxyethyl)-benzol
Ausbeute: 75 % der Theorie
Masspektrum: MH⁺ 229 (100)

### 2.41. 2-Hydroxy-5-amino-4'-(1-hydroxy-ethyl)-biphenyl Hydrochlorid

Verwendete Bromderivat: 1-Brom-4-(1-Hydroxyethyl)-benzol
Ausbeute: 56 % der Theorie
Masspektrum: MH⁺ 229 (100)

### Beispiele 3 bis 44: Haarfärbemittel

Es werden Haarfärbelösungen der folgenden Zusammensetzung hergestellt:

| | |
|---|---|
| 1,25 mmol | Entwicklersubstanz der Formel (I) gemäss Tabelle 1 |
| 1,25 mmol | Kupplersubstanz gemäss Tabelle 1 |
| 1,0 g | Kaliumoleat (8prozentige wässrige Lösung) |
| 1,0 g | Ammoniak (22prozentige wässrige Lösung) |
| 1,0 g | Ethanol |
| 0,3 g | Ascorbinsäure |
| ad 100,0 g | Wasser |

50 g der vorstehenden Färbelösung werden unmittelbar vor der Anwendung mit 50 g einer 6prozentigen wässrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefaßt.

**Tabelle 1:**

| **Beispiel** | **Entwickler-substanz der Formel (I)** | **Kupplersubstanz** | | | |
|---|---|---|---|---|---|
| | | **I.** **1,3-Di- hydroxybenzol** | **II.** **1,3-Diamino-4-(2-hydroxyethoxy)-benzol*sulfat** | **III.** **5-Amino-2-methylphenol** | **IV.** **1-Naphtol** |
| **3.** | **1D** | hellblond | rotviolett | rotorange | violett |
| **4.** | **2.1.** | hellblond | rotviolett | rotorange | violett |
| **5.** | **2.2.** | hellblond | hellviolett | hellorange | hellviolett |
| **6.** | **2.3.** | hellblond | hellviolett | hellorange | hellviolett |
| **7.** | **2.4.** | hellblond | hellviolett | hellorange | hellviolett |
| **8.** | **2.5.** | hellblond | hellviolett | hellorange | hellviolett |
| **9.** | **2.6.** | hellblond | hellviolett | hellorange | hellviolett |
| **10.** | **2.7.** | hellblond | hellviolett | hellorange | hellviolett |
| **11.** | **2.8.** | hellblond | hellviolett | hellorange | hellviolett |
| **12.** | **2.9.** | hellblond | hellviolett | hellorange | hellviolett |
| **13.** | **2.10.** | hellblond | hellviolett | hellorange | hellviolett |
| **14.** | **2.11.** | hellblond | hellviolett | hellorange | hellviolett |
| **15.** | **2.12.** | hellblond | hellviolett | hellorange | hellviolett |
| **16.** | **2.13.** | hellblond | hellviolett | hellorange | hellviolett |
| **17.** | **2.14.** | hellblond | hellviolett | hellorange | hellviolett |
| **18.** | **2.15.** | hellblond | hellviolett | hellorange | hellviolett |
| **19.** | **2.16.** | hellblond | hellviolett | hellorange | hellviolett |
| **20.** | **2.17.** | hellblond | hellviolett | hellorange | hellviolett |
| **21.** | **2.18.** | hellblond | hellviolett | hellorange | hellviolett |
| **22.** | **2.19.** | hellblond | hellviolett | hellorange | hellviolett |
| **23.** | **2.20.** | hellblond | hellviolett | hellorange | hellviolett |
| **24.** | **2.21.** | hellblond | hellviolett | hellorange | hellviolett |
| **25.** | **2.22.** | hellblond | hellviolett | hellorange | hellviolett |
| **26.** | **2.23.** | hellblond | hellviolett | hellorange | hellviolett |
| **27.** | **2.24.** | hellblond | hellviolett | hellorange | hellviolett |
| **28.** | **2.25.** | hellblond | hellviolett | hellorange | hellviolett |
| **29.** | **2.26.** | hellblond | hellviolett | hellorange | hellviolett |
| **30.** | **2.27.** | hellblond | hellviolett | hellorange | hellviolett |
| **31.** | **2.28.** | hellblond | hellviolett | hellorange | hellviolett |
| **32.** | **2.29.** | hellblond | hellviolett | hellorange | hellviolett |
| **33.** | **2.30.** | hellblond | hellviolett | hellorange | hellviolett |
| **34.** | **2.31.** | hellblond | hellviolett | hellorange | hellviolett |
| **35.** | **2.32.** | hellblond | hellviolett | hellorange | hellviolett |
| **36.** | **2.33.** | hellblond | hellviolett | hellorange | hellviolett |
| **37.** | **2.34.** | hellblond | hellviolett | hellorange | hellviolett |
| **38.** | **2.35.** | hellblond | hellviolett | hellorange | hellviolett |
| **39.** | **2.36.** | hellblond | hellviolett | hellorange | hellviolett |
| **40.** | **2.37.** | hellblond | hellviolett | hellorange | hellviolett |
| **41.** | **2.38.** | hellblond | hellviolett | hellorange | hellviolett |
| **42.** | **2.39.** | hellblond | hellviolett | hellorange | hellviolett |
| **43.** | **2.40.** | hellblond | hellviolett | hellorange | hellviolett |
| **44.** | **2.41.** | hellblond | hellviolett | hellorange | hellviolett |

### Beispiele 45 bis 60: Haarfärbemittel

Es werden Haarfärbelösungen der folgenden Zusammensetzung hergestellt:

| | |
|---|---|
| X g | Entwicklersubstanz E1 der Formel (I) gemäss Tabelle 2 |
| U g | Entwicklersubstanz E2 bis E9 gemäss Tabelle 2 |
| Y g | Kupplersubstanz K11 bis K36 gemäss Tabelle 4 |
| Z g | direktziehender Farbstoff D1 bis D3 gemäss Tabelle 3 |
| 10,0 g | Kaliumoleat (8prozentige wässrige Lösung) |
| 10,0 g | Ammoniak (22prozentige wässrige Lösung) |
| 10,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen wässsrigen Wasserstoffperoxidlösung vermischt. Anschliessend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die Färbeergebnisse sind in Tabelle 5 zusammengefasst.

### Beispiele 61 bis 66: Haarfärbemittel

Es werden cremeförmige Farbträgermassen der folgenden Zusammensetzung hergestellt:

| | |
|---|---|
| X g | Entwicklersubstanz E1 der Formel (I) gemäss Tabelle 2 |
| Y g | Kupplersubstanz K11 bis K36 gemäss Tabelle 4 |
| Z g | direktziehender Farbstoff D2 gemäss Tabelle 3 |
| 15,0 g | Cetylalkohol |
| 0,3 g | Ascorbinsäure |
| 3,5 g | Natriumlaurylalkoholdiglycolethersulfat, 28%ige wässrige Lösung |
| 3,0 g | Ammoniak, 22%ige wässrige Lösung |
| 0,3 g | Natriumsulfit, wasserfrei |
| ad 100 g | Wasser |

40 g der vorstehenden Färbecreme werden unmittelbar vor der Anwendung mit 40 g einer 6prozentigen Wasserstoffperoxidlösung vermischt. Anschliessend wird das Gemisch auf das Haar aufgetragen. Nach einer Einwirkzeit von 30 Minuten wird das Haar mit Wasser gespült, mit einem handelsüblichen Shampoo gewaschen und getrocknet. Die Färbeergebnisse sind der nachfolgenden Tabelle 6 zu entnehmen.

**Tabelle 2:**

| **Entwicklersubstanzen** | |
|---|---|
| **E1** | 2-Hydroxy-5-amino-biphenyl Hydrochlorid (gemäss Beispiel **1D**) |
| | |
| **E2** | 1,4-Diaminobenzol |
| **E3** | 2,5-Diamino-phenylethanol-sulfat |
| **E4** | 3-Methyl-4-amino-phenol |
| **E5** | 4-Amino-2-aminomethyl-phenol-dihydrochlorid |
| **E6** | 4-Amino-phenol |
| **E7** | N,N-Bis(2'-hydroxyethyl)-p-phenylendiamin-sulfat |
| **E8** | 4,5-Diamino-1-(2'-hydroxyethyl)-pyrazol-sulfat |
| **E9** | 2,5-Diaminotoluol-sulfat |

**Tabelle 3:**

| **Direktziehende Farbstoffe** | |
|---|---|
| **D1** | 2,6-Diamino-3-((pyridin-3-yl)azo)pyridin |
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K11** | 1,3-Diaminobenzol |
| **K12** | 2-Amino-4-(2'-hydroxyethyl)amino-anisol-sulfat |
| **K13** | 1,3-Diamino-4-(2'-Hydroxyethoxy)benzol-sulfat |
| **K14** | 2,4-Diamino-5-fluor-toluol-sulfat |
| **K15** | 3-Amino-2-methylamino-6-methoxy-pyridin |
| **K16** | 3,5-Diamino-2,6-dimethoxy-pyridin-dihydrochlorid |
| **K17** | 2,4-Diamino-5-ethoxy-toluol-sulfat |
| **K18** | N-(3-Dimethylamino)phenylhamstoff |
| **K19** | 1,3-Bis(2,4-Diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzolhydrochlorid |
| K35 | 3,4-Methylendioxy-phenol |
| **K36** | 2-Amino-5-methyl-phenol |

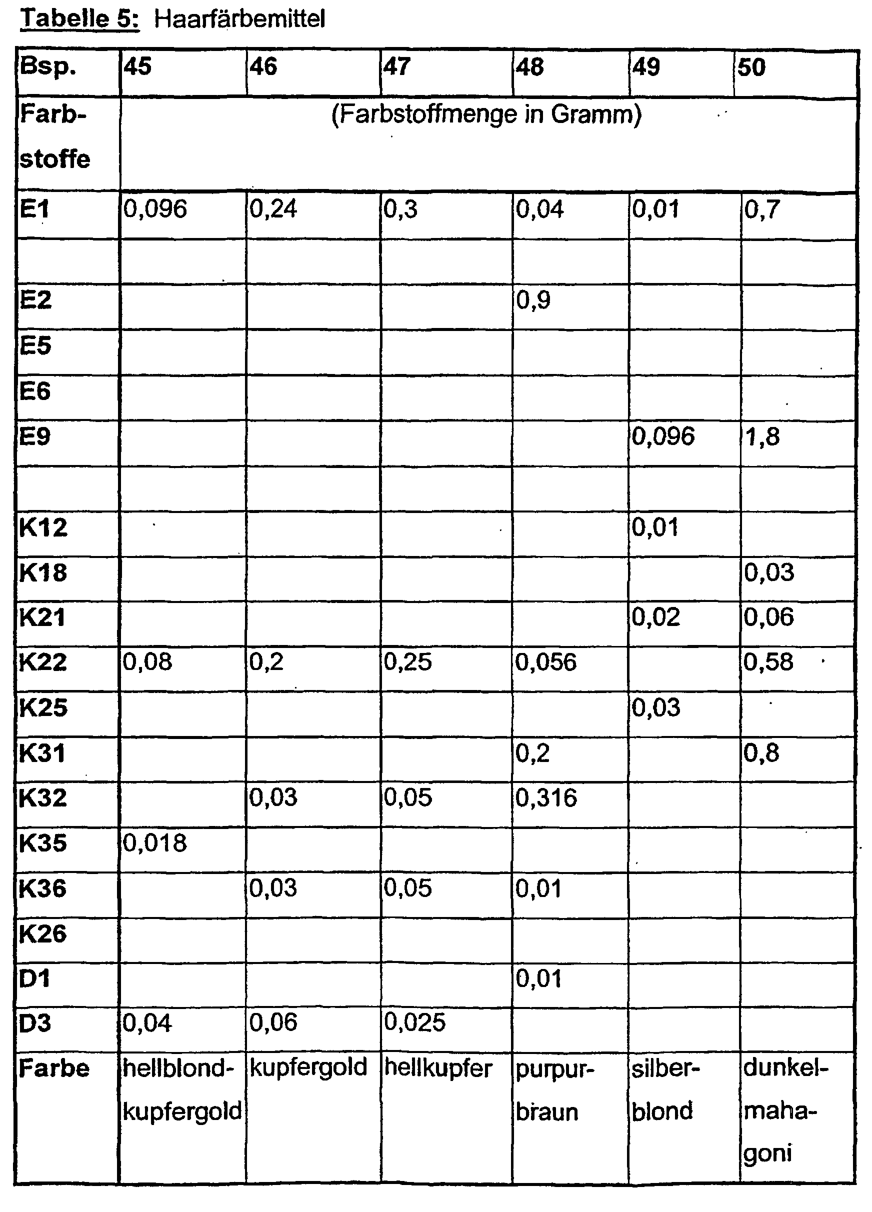

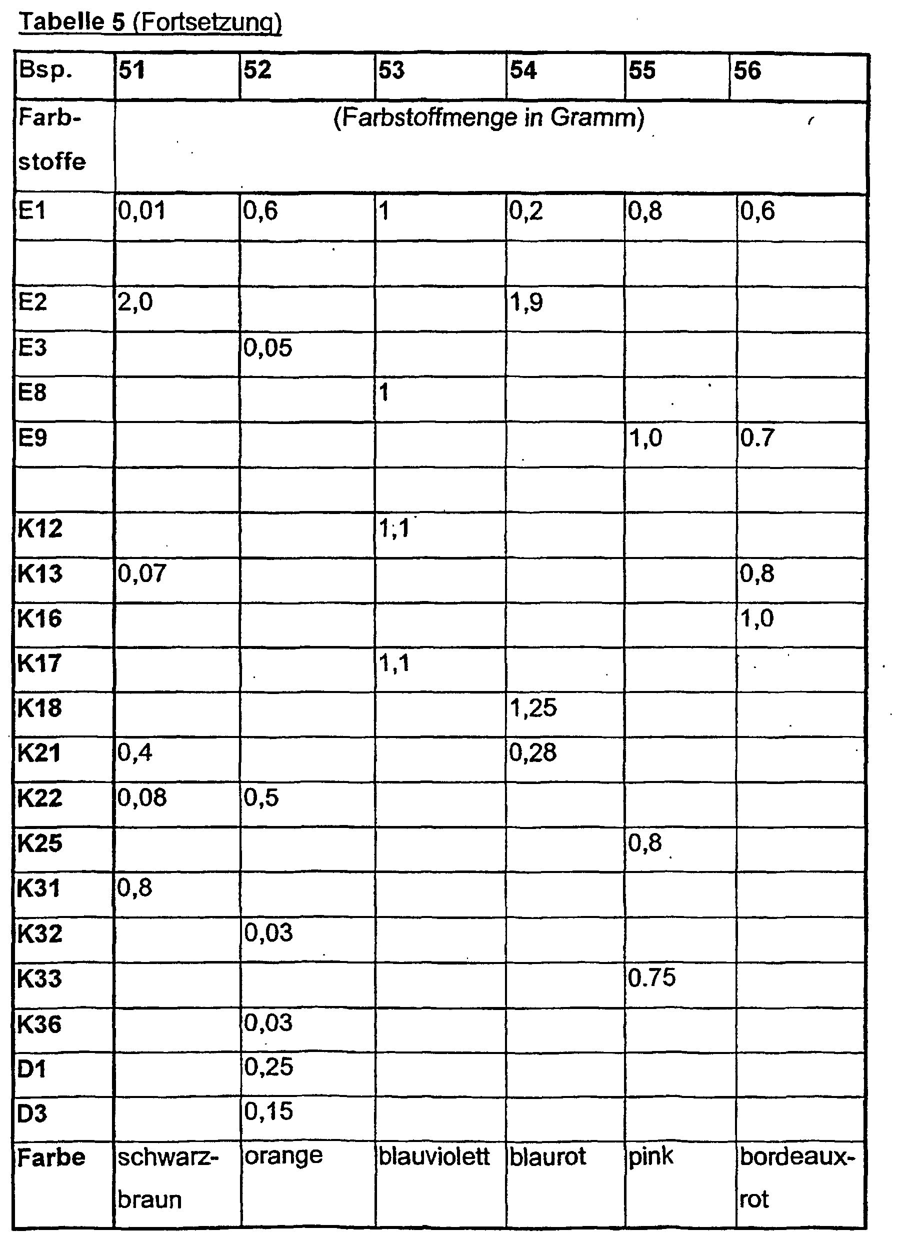

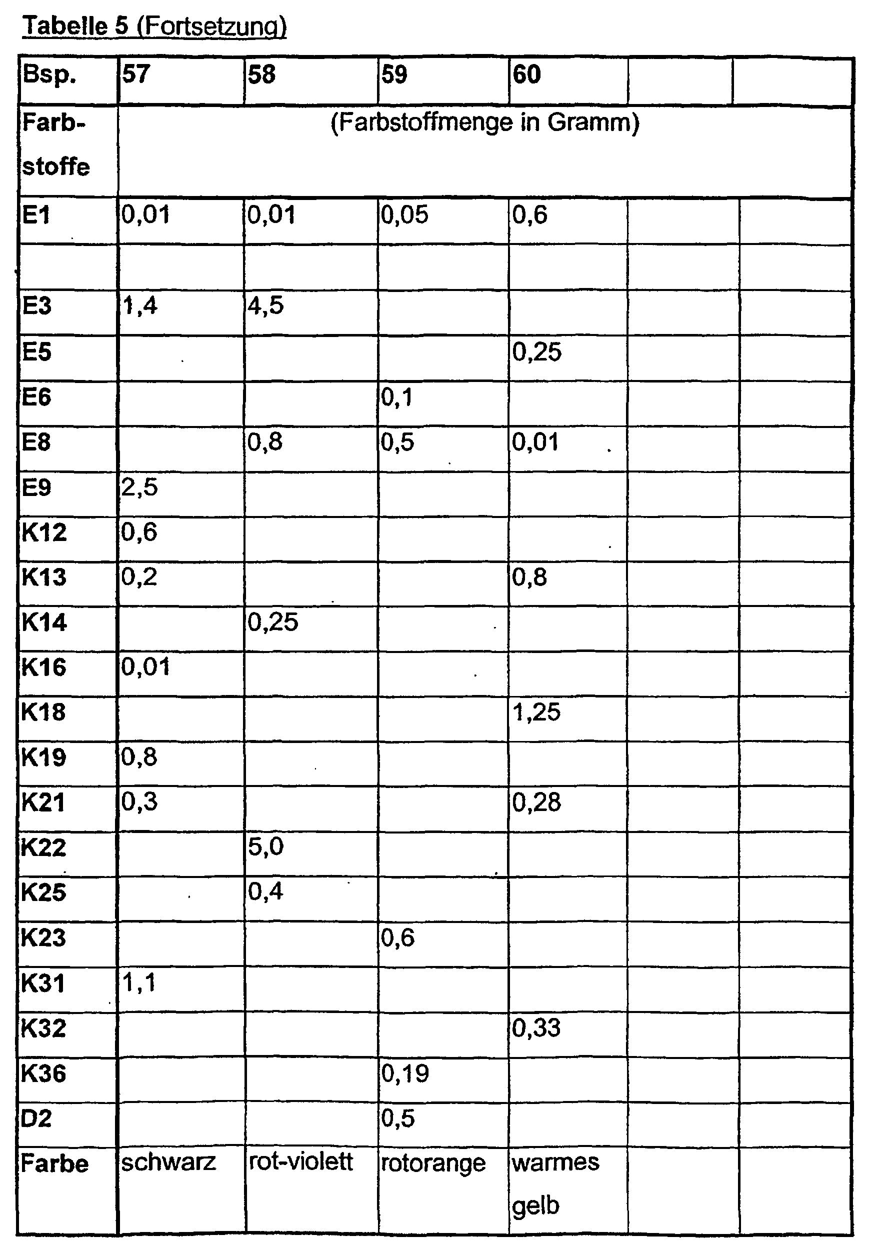

**Tabelle 6:**

| Haarfärbemittel | | | | | | |
|---|---|---|---|---|---|---|
| **Bsp.** | **61** | **62** | **63** | **64** | **65** | **66** |
| **Farbstoffe** | (Farbstoffmenge in Gramm) | | | | | |
| **E1** | 0,1 | 0,2 | 0,01 | 2,0 | 0,5 | 0,7 |
| | | | | | | |
| **E4** | | | | | | 1,6 |
| **E8** | | | | 0,25 | 0,8 | 0,2 |
| **E9** | 3,2 | 1,71 | 0,02 | | | 1,8 |
| | | | | | | |
| **K13** | 0,23 | 0,1 | | | 1,3 | |
| **K14** | 0,2 | | | | | |
| **K16** | | | 0,015 | | | |
| **K21** | 0,4 | 0,8 | | | 0,02 | |
| **K22** | 0,08 | | 0,25 | 1,8 | | 4,5 |
| **K23** | | 0,2 | | | 0,03 | |
| **K31** | 1,05 | 0,135 | 0,02 | 0,25 | | 0,8 |
| **K25** | | | | | | 0,55 |
| **K26** | | | 0,03 | | | |
| **K19** | | | | | 1,7 | |
| **K36** | | 0,27 | | | | |
| **D2** | | 0,01 | | | | |
| **Farbe** | dunkelbraun | schokobraun | silberblond | orange | blauviolett | rotviolett |

Alle in der vorliegenden Anmeldung enthaltenen Prozentangaben stellen soweit nicht anders angegeben Gewichtsprozente dar.

## Patentansprüche

1. Mittel zur oxidativen Färbung von Keratinfasem, insbesondere menschlichen Haaren, auf der Basis einer Entwicklersubstanz-Kupplersubstanz-Kombination, **dadurch gekennzeichnet, dass** es als Entwicklersubstanz mindestens ein 2-Hydroxy-5-amino-biphenyl-Derivat aus der Gruppe bestehend aus 2-Hydroxy-3-chlor-5-amino-biphenyl; 2-Hydroxy-3-methyl-5-amino-biphenyl; 2-Hydroxy-5-amino-biphenyl; 2-Hydroxy-4-chlor-5-amino-biphenyl; 2-Hydroxy-4-methyl-5-amino-biphenyl; 2,2'-Dihydroxy-5-amino-biphenyl; 2,3'-Dihydroxy-5-amino-biphenyl; 2,4'-Dihydroxy-5-amino-biphenyl; 2,5'-Dihydroxy-5-amino-biphenyl; 2,6'-Dihydroxy-5-amino-biphenyl; 2-Hydroxy-5,2'-diamino-biphenyl; 2-Hydroxy-5,3'-diamino-biphenyl; 2-Hydroxy-5,4'-diamino-biphenyl; 2-Hydroxy-5,5'-diamino-biphenyl; 2-Hydroxy-5,6'-diamino-biphenyl; 2,2',3'-Trihydroxy-5-amino-biphenyl; 2,2',4'-Trihydroxy-5-amino-biphenyl; 2,2',5'-Trihydroxy-5-amino-biphenyl; 2,2',6'-Trihydroxy-5-amino-biphenyl; 2,3',4'-Trihydroxy-5-amino-biphenyl; 2,3',5'-Trihydroxy-5-amino-biphenyl; 2-Hydroxy-5,2',3'-triamino-biphenyl; 2-Hydroxy-5,2',4-triamino-biphenyl; 2-Hydroxy-5,2',5'-triamino-biphenyl; 2-Hydroxy-5,2',6'-triamino-biphenyl; 2-Hydroxy-5,3',4'-triamino-biphenyl; 2-Hydroxy-5,3',5'-triamino-biphenyl; 2-Hydroxy-5,3',4'-triamino-biphenyl; 2-Hydroxy-5,3',5'-triamino-biphenyl; 2-Hydroxy-5-amino-2'-aminomethyl-biphenyl; 2-Hydroxy-5-amino-2'-chlor-biphenyl; 2-Hydroxy-5-amino-2'-cyan-biphenyl; 2-Hydroxy-5-amino-2'-fluor-biphenyl; 2-Hydroxy-5-amino-2'-methoxy-biphenyl; 2-Hydroxy-5-amino-2'-methyl-biphenyl; 2-Hydroxy-5-amino-2'-methylsulfanyl-biphenyl; 2-Hydroxy-5-amino-2'-nitro-biphenyl; 2-Hydroxy-5-amino-3'-aminomethyl-biphenyl; 2-Hydroxy-5-amino-3'-chlor-biphenyl; 2-Hydroxy-5-amino-3'-cyan-biphenyl; 2-Hydroxy-5-amino-3'-fluor-biphenyl; 2-Hydroxy-5-amino-3'-methoxy-biphenyl; 2-Hydroxy-5-amino-3'-methyl-biphenyl; 2-Hydroxy-5-amino-3'-methylsulfanyl-biphenyl; 2-Hydroxy-5-amino-3'-nitro-biphenyl; 2-Hydroxy-5-amino-4'-aminomethyl-biphenyl; 2-Hydroxy-5-amino-4'-chlor-biphenyl; 2-Hydroxy-5-amino-4'-cyan-biphenyl; 2-Hydroxy-5-amino-4'-fluor-biphenyl; 2-Hydroxy-5-amino-4'-methoxy-biphenyl; 2-Hydroxy-5-amino-4'-methyl-biphenyl; 2-Hydroxy-5-amino-4'-methylsulfanyl-biphenyl; 2-Hydroxy-5-amino-4'-nitro-biphenyl; 2-Hydroxy-5-amino-5'-aminomethyl-biphenyl; 2-Hydroxy-5-amino-5'-chlor-biphenyl; 2-Hydroxy-5-amino-5'-cyan-biphenyl; 2-Hydroxy-5-amino-5'-fluor-biphenyl; 2-Hydroxy-5-amino-5'-methoxy-biphenyl; 2-Hydroxy-5-amino-5'-methyl-biphenyl; 2-Hydroxy-5-amino-5'-methylsulfanyl-biphenyl; 2-Hydroxy-5-amino-5'-nitro-biphenyl; 2-Hydroxy-5-amino-6'-aminomethyl-biphenyl; 2-Hydroxy-5-amino-6'-chlor-biphenyl; 2-Hydroxy-5-amino-6'-cyan-biphenyl; 2-Hydroxy-5-amino-6'-fluor-biphenyl; 2-Hydroxy-5-amino-6'-methoxy-biphenyl; 2-Hydroxy-5-amino-6'-methyl-biphenyl; 2-Hydroxy-5-amino-6'-methylsulfanyl-biphenyl; 2-Hydroxy-5-amino-6'-nitro-biphenyl; 2,2'-Dihydroxy-5-amino-3'-methyl-biphenyl; 2,2'-Dihydroxy-5-amino-4'-methyl-biphenyl; 2,2'-Dihydroxy-5-amino-5'-methyl-biphenyl; 2,2'-Dihydroxy-5-amino-6'-methyl-biphenyl; 2,3-Dihydroxy-5-amino-4-methyl-biphenyl; 2,3'-Dihydroxy-5-amino-5'-methyl-biphenyl; 2-Hydroxy-5-amino-2',3'-dimethoxy-biphenyl; 2-Hydroxy-5-amino-2',3'-dimethyl-biphenyl; 2-Hydroxy-5-amino-2',4'-dimethoxy-biphenyl; 2-Hydroxy-5-amino-2',4'-dimethyl-biphenyl; 2-Hydroxy-5-amino-2',5'-dimethoxy-biphenyl; 2-Hydroxy-5-amino-2',5'-dimethyl-biphenyl; 2-Hydroxy-5-amino-2',6'-dimethoxy-biphenyl; 2-Hydroxy-5-amino-2',6'-dimethyl-biphenyl; 2-Hydroxy-5-amino-3',4'-dimethoxy-biphenyl; 2-Hydroxy-5-amino-3',4'-dimethyl-biphenyl; 2-Hydroxy-5-amino-3',5'-dimethoxy-biphenyl; 2-Hydroxy-5-amino-3',5'-dimethyl-biphenyl; 2-Hydroxy-5-amino-2'-methoxy-3'-methyl-biphenyl; 2-Hydroxy-5-amino-2'-methoxy-4'-methyl-biphenyl; 2-Hydroxy-5-amino-2'-methoxy-5'-methyl-biphenyl; 2-Hydroxy-5-amino-2'-methoxy-6'-methyl-biphenyl; 2-Hydroxy-5-amino-3'-methoxy-4'-methyl-biphenyl; 2-Hydroxy-5-amino-3'-methoxy-5'-methyl-biphenyl; 4-Amino-2-benzo[1,3]dioxol-5-yl-phenol; 4-Amino-2-benzo[2,4]dioxol-5-yl-phenol und 2-Hydroxy-5-amino-4'-(2-hydroxy-ethoxy)-biphenyl oder deren physiologisch verträglichen, wasserlöslichen Salzen,enthält.

2. Mittel nach Anspruch 1, **dadurch gekennzeichnet, dass** das 2-Hydroxy-5-amino-biphenyl-Derivat ausgewählt ist aus 2-Hydroxy-5-amino-biphenyl; 2,4'-Dihydroxy-5-amino-biphenyl, 2-Hydroxy-5-amino-4'-(2"-hydroxyethoxy)-biphenyl, 2,4'-Dihydroxy-5-amino-2'-methyl-biphenyl, 2-Hydroxy-5-amino-4'-(2"-hydroxyethyl)-biphenyl, 2-Hydroxy-5,4'-diamino-biphenyl oder deren physiologisch verträglichen Salzen.

3. Mittel nach Anspruch 1 oder 2, **dadurch gekennzeichnet, dass** das 2-Hydroxy-5-amino-biphenyl-Derivat in einer Menge von 0,005 bis 20,0 Gewichtsprozent enthalten ist.

4. Mittel einem der Ansprüche 1 bis 3, **dadurch gekennzeichnet, dass** es einen pH-Wert von 6,5 bis 11,5 aufweist.

5. 2-Hydroxy-5-amino-biphenyl-Derivate welche ausgewählt sind aus 2-Hydroxy-3-chlor-5-amino-biphenyl; 2-Hydroxy-3-methyl-5-amino-biphenyl; 2-Hydroxy-4-chlor-5-amino-biphenyl; 2-Hydroxy-4-methyl-5-amino-biphenyl; 2,2'-Dihydroxy-5-amino-biphenyl; 2,3'-Dihydroxy-5-amino-biphenyl; 2,4'-Dihydroxy-5-amino-biphenyl; 2,5'-Dihydroxy-5-amino-biphenyl; 2,6'-Dihydroxy-5-amino-biphenyl; 2-Hydroxy-5,2'-diamino-biphenyl; 2-Hydroxy-5,3'-diamino-biphenyl; 2-Hydroxy-5,4'-diamino-biphenyl; 2-Hydroxy-5,5'-diamino-biphenyl; 2-Hydroxy-5,6'-diamino-biphenyl; 2,2',3'-Trihydroxy-5-amino-biphenyl; 2,2',4'-Trihydroxy-5-amino-biphenyl; 2,2',5'-Trihydroxy-5-amino-biphenyl; 2,2',6'-Trihydroxy-5-amino-biphenyl; 2,3',4'-Trihydroxy-5-amino-biphenyl; 2,3',5'-Trihydroxy 5-amino-biphenyl; 2-Hydroxy-5,2',3'-triamino-biphenyl; 2-Hydroxy-5,2',4'-triamino-biphenyl; 2-Hydroxy-5,2',5'-triamino-biphenyl; 2-Hydroxy-5,2',6-triamino-biphenyl; 2-Hydroxy-5,3',4'-triamino-biphenyl; 2-Hydroxy-5,3',5'-triamino-biphenyl; 2-Hydroxy-5,3',4'-triamino-biphenyl; 2-Hydroxy-5,3',5-triamino-biphenyl; 2-Hydroxy-5-amino-2'-aminomethyl-biphenyl; 2-Hydroxy-5-amino-2'-chlor-biphenyl; 2-Hydroxy-5-amino-2'-cyan-biphenyl; 2-Hydroxy-5-amino-2'-fluor-biphenyl; 2-Hydroxy-5-amino-2'-methoxy-biphenyl; 2-Hydroxy-5-amino-2'-methyl-biphenyl; 2-Hydroxy-5-amino-2'-methylsulfanyl-biphenyl; 2-Hydroxy-5-amino-2'-nitro-biphenyl; 2-Hydroxy-5-amino-3'-aminomethyl-biphenyl; 2-Hydroxy-5-amino-3'-chlor-biphenyl; 2-Hydroxy-5-amino-3'-cyan-biphenyl; 2-Hydroxy-5-amino-3'-fluor-biphenyl; 2-Hydroxy-5-amino-3'-methoxy-biphenyl; 2-Hydroxy-5-amino-3'-methyl-biphenyl; 2-Hydroxy-5-amino-3'-methylsulfanyl-biphenyl; 2-Hydroxy-5-amino-3'-nitro-biphenyl; 2-Hydroxy-5-amino-4-aminomethyl-biphenyl; 2-Hydroxy-5-amino-4'-cyan-biphenyl; 2-Hydroxy-5-amino-4'-fluor-biphenyl; 2-Hydroxy-5-amino-4'-methoxy-biphenyl; 2-Hydroxy-5-amino-4'-methyl-biphenyl; 2-Hydroxy-5-amino-4'-methylsulfanyl-biphenyl; 2-Hydroxy-5-amino-4'-nitro-biphenyl; 2-Hydroxy-5-amino-5'-aminomethyl-biphenyl; 2-Hydroxy-5-amino-5'-chlor-biphenyl; 2-Hydroxy-5-amino-5-cyan-biphenyl; 2-Hydroxy-5-amino-5'-fluor-biphenyl; 2-Hydroxy-5-amino-5'-methoxy-biphenyl; 2-Hydroxy-5-amino-5'-methyl-biphenyl; 2-Hydroxy-5-amino-5'-methylsulfanyl-biphenyl; 2-Hydroxy-5-amino-5'-nitro-biphenyl; 2-Hydroxy-5-amino-6'-aminomethyl-biphenyl; 2-Hydroxy-5-amino-6'-chlor-biphenyl; 2-Hydroxy-5-amino-6'-cyan-biphenyl; 2-Hydroxy-5-amino-6'-fluor-biphenyl; 2-Hydroxy-5-amino-6'-methoxy-biphenyl; 2-Hydroxy-5-amino-6'-methyl-biphenyl; 2-Hydroxy-5-amino-6'-methylsulfanyl-biphenyl; 2-Hydroxy-5-amino-6'-nitro-biphenyl; 2,2'-Dihydroxy-5-amino-3'-methyl-biphenyl; 2,2'-Dihydroxy-5-amino-4'-methyl-biphenyl; 2,2'-Dihydroxy 5-amino-5'-methyl-biphenyl; 2,2'-Dihydroxy-5-amino-6'-methyl-biphenyl; 2,3'-Dihydroxy-5-amino-4'-methyl-biphenyl; 2,3'-Dihydroxy-5-amino-5'-methyl-biphenyl; 2-Hydroxy-5-amino-2',3'-dimethoxy-biphenyl; 2-Hydroxy-5-amino-2',3'-dimethyl-biphenyl; 2-Hydroxy-5-amino-2',4'-dimethoxy-biphenyl; 2-Hydroxy-5-amino-2',4'-dimethyl-biphenyl; 2-Hydroxy-5-amino-2',5'-dimethoxy-biphenyl; 2-Hydroxy-5-amino-2',5-dimethyl-biphenyl; 2-Hydroxy-5-amino-2',6'-dimethoxy-biphenyl; 2-Hydroxy-5-amino-2',6'-dimethyl-biphenyl; 2-Hydroxy-5-amino-3',4'-dimethoxy-biphenyl; 2-Hydroxy-5-amino-3',4'-dimethyl-biphenyl; 2-Hydroxy-5-amino-3',5'-dimethoxy-biphenyl; 2-Hydroxy-5-amino-3',5'-dimethyl-biphenyl; 2-Hydroxy-5-amino-2'-methoxy-3'-methyl-biphenyl; 2-Hydroxy-5-amino-2'-methoxy-4'-methyl-biphenyl; 2-Hydroxy-5-amino-2'-methoxy-5'-methyl-biphenyl; 2-Hydroxy-5-amino-2'-methoxy-6'-methyl-biphenyl; 2-Hydroxy-5-amino-3'-methoxy-4'-methyl-biphenyl; 2-Hydroxy-5-amino-3'-methoxy-5'-methyl-biphenyl, 4-Amino-2-benzo[1,3]dioxol-5-yl-phenol; 4-Amino-2-benzo[2,4]dioxol-5-yl-phenol und 2-Hydroxy-5-amino-4'-(2-hydroxyethoxy)-biphenyl oder deren physiologisch verträglichen, wasserlöslichen Salzen.

6. Verbindung nach Anspruch 5, **dadurch gekennzeichnet, dass** sie ausgewählt ist aus 2,4'-Dihydroxy-5-amino-biphenyl, 2-Hydroxy-5-amino-4'-(2"-hydroxyethoxy)-biphenyl, 2,4'-Dihydroxy-5-amino-2'-methyl-biphenyl, 2-Hydroxy-5-amino-4'-(2"-hydroxyethyl)-biphenyl, 2-Hydroxy-5,4'-diamino-biphenyl oder deren physiologisch verträglichen Salzen.

## Claims

1. Agent for the oxidative colouring of keratin fibres, in particular human hair, based on a developer substance - coupler substance combination, **characterized in that** it comprises, as developer substance, at least one 2-hydroxy-5-aminobiphenyl derivative from the group consisting of 2-hydroxy-3-chloro-5-aminobiphenyl; 2-hydroxy-3-methyl-5-aminobiphenyl; 2-hydroxy-5-aminobiphenyl; 2-hydroxy-4-chloro-5-aminobiphenyl; 2-hydroxy-4-methyl-5-aminobiphenyl; 2,2'-dihydroxy-5-aminobiphenyl; 2,3'-dihydroxy-5-aminobiphenyl; 2,4'-dihydroxy-5-aminobiphenyl; 2,5'-dihydroxy-5-aminobiphenyl; 2,6'-dihydroxy-5-aminobiphenyl; 2-hydroxy-5,2'-diaminobiphenyl; 2-hydroxy-5,3'-diaminobiphenyl; 2-hydroxy-5,4'-diaminobiphenyl; 2-hydroxy-5,5'-diaminobiphenyl; 2-hydroxy-5,6'-diaminobiphenyl; 2,2',3'-trihydroxy-5-aminobiphenyl; 2,2',4'-trihydroxy-5-aminobiphenyl; 2,2',5'-trihydroxy-5-aminobiphenyl; 2,2',6'-trihydroxy-5-aminobiphenyl; 2,3',4'-trihydroxy-5-aminobiphenyl; 2,3',5'-trihydroxy-5-aminobiphenyl; 2-hydroxy-5,2',3'-triaminobiphenyl; 2-hydroxy-5,2',4'-triaminobiphenyl; 2-hydroxy-5,2',5'-triaminobiphenyl; 2-hydroxy-5,2',6'-triaminobiphenyl; 2-hydroxy-5,3',4'-triaminobiphenyl; 2-hydroxy-5,3',5'-triaminobiphenyl; 2-hydroxy-5,3',4'-triaminobiphenyl; 2-hydroxy-5,3',5'-triaminophenyl; 2-hydroxy-5-amino-2'-aminomethylbiphenyl; 2-hydroxy-5-amino-2'-chlorobiphenyl; 2-hydroxy-5-amino-2'-cyanobiphenyl; 2-hydroxy-5-amino-2'-fluorobiphenyl; 2-hydroxy-5-amino-2'-methoxybiphenyl; 2-hydroxy-5-amino-2'-methylbiphenyl; 2-hydroxy-5-amino-2'-methylsulfanylbiphenyl; 2-hydroxy-5-amino-2'-nitrobiphenyl; 2-hydroxy-5-amino-3'-aminomethyl-biphenyl; 2-hydroxy-5-amino-3'-chlorobiphenyl; 2-hydroxy-5-amino-3'-cyanobiphenyl; 2-hydroxy-5-amino-3'-fluorobiphenyl; 2-hydroxy-5-amino-3'-methoxybiphenyl; 2-hydroxy-5-amino-3'-methylbiphenyl, 2-hydroxy-5-amino-3'-methylsulfanylbiphenyl; 2-hydroxy-5-amino-3'-nitrobiphenyl; 2-hydroxy-5-amino-4'-aminomethylbiphenyl; 2-hydroxy-5-amino-4'-chlorobiphenyl; 2-hydroxy-5-amino-4'-cyanobiphenyl; 2-hydroxy-5-amino-4'-fluorobiphenyl; 2-hydroxy-5-amino-4'-methoxybiphenyl; 2-hydroxy-5-amino-4'-methylbiphenyl; 2-hydroxy-5-amino-4'-methylsulfanylbiphenyl; 2-hydroxy-5-amino-4'-nitrobiphenyl; 2-hydroxy-5-amino-5'-aminomethylbiphenyl; 2-hydroxy-5-amino-5'-chlorobiphenyl; 2-hydroxy-5-amino-5'-cyanobiphenyl; 2-hydroxy-5-amino-5'-fluorobiphenyl; 2-hydroxy-5-amino-5'-methoxybiphenyl; 2-hydroxy-5-amino-5'-methylbiphenyl; 2-hydroxy-5-amino-5'-methylsulfanylbiphenyl; 2-hydroxy-5-amino-5'-nitrobiphenyl; 2-hydroxy-5-amino-6'-aminomethylbiphenyl; 2-hydroxy-5-amino-6'-chlorobiphenyl; 2-hydroxy-5-amino-6'-cyanobiphenyl; 2-hydroxy-5-amino-6'-fluorobiphenyl; 2-hydroxy-5-amino-6'-methoxybiphenyl; 2-hydroxy-5-amino-6'-methylbiphenyl; 2-hydroxy-5-amino-6'-methylsulfanylbiphenyl; 2-hydroxy-5-amino-6'-nitrobiphenyl; 2,2'-dihydroxy-5-amino-3'-methylbiphenyl; 2,2'-dihydroxy-5-amino-4'-methylbiphenyl; 2,2'-dihydroxy-5-amino-5'-methylbiphenyl; 2,2'-dihydroxy-5-amino-6'-methylbiphenyl; 2,3'-dihydroxy-5-amino-4'-methylbiphenyl; 2,3'-dihydroxy-5-amino-5'-methylbiphenyl; 2-hydroxy-5-amino-2',3'-dimethoxybiphenyl; 2-hydroxy-5-amino-2',3'-dimethylbiphenyl; 2-hydroxy-5-amino-2',4'-dimethoxybiphenyl; 2-hydroxy-5-amino-2',4'-dimethylbiphenyl; 2-hydroxy-5-amino-2',5'-dimethoxybiphenyl; 2-hydroxy-5-amino-2',5'-dimethylbiphenyl; 2-hydroxy-5-amino-2',6'-dimethoxybiphenyl; 2-hydroxy-5-amino-2'6'-dimethylbiphenyl; 2-hydroxy-5-amino-3',4'-dimethoxybiphenyl; 2-hydroxy-5-amino-3'4'-dimethylbiphenyl; 2-hydroxy-5-amino-3',5'-dimethoxybiphenyl; 2-hydroxy-5-amino-3',5'dimethylbiphenyl; 2-hydroxy-5-amino-2'-methoxy-3'-methylbiphenyl; 2-hydroxy-5-amino-2'-methoxy-4'-methylbiphenyl; 2-hydroxy-5-amino-2'-methoxy-5'-methylbiphenyl; 2-hydroxy-5-amino-2'-methoxy-6'-methylbiphenyl; 2-hydroxy-5-amino-3'-methoxy-4'-methylbiphenyl; 2-hydroxy-5-amino-3'-methoxy-5'-methylbiphenyl; 4-amino-2-benzo[1,3]dioxol-5-yl-phenol; 4-amino-2-benzo[2,4]dioxol-5-yl-phenol and 2-hydroxy-5-amino-4'-(2-hydroxyethoxy)biphenyl or physiologically compatible, water-soluble salts thereof.

2. Agent according to Claim 1, **characterized in that** the 2-hydroxy-5-aminobiphenyl derivative is chosen from 2-hydroxy-5-aminobiphenyl; 2,4'-dihydroxy-5-aminobiphenyl, 2-hydroxy-5-amino-4'-(2"-hydroxyethoxy)biphenyl, 2,4'-dihydroxy-5-amino-2'-methylbiphenyl, 2-hydroxy-5-amino-4'-(2"-hydroxyethyl)biphenyl, 2-hydroxy-5,4'-diaminobiphenyl or physiologically compatible salts thereof.

3. Agent according to Claim 1 or 2, **characterized in that** the 2-hydroxy-5-aminobiphenyl derivative is present in an amount of from 0.005 to 20.0% by weight.

4. Agent according to one of Claims 1 to 3,
**characterized in that** it has a pH of from 6.5 to 11.5.

5. 2-hydroxy-5-aminobiphenyl derivatives which are chosen from 2-hydroxy-3-chloro-5-aminobiphenyl; 2-hydroxy-3-methyl-5-aminobiphenyl; 2-hydroxy-4-chloro-5-aminobiphenyl; 2-hydroxy-4-methyl-5-aminobiphenyl; 2,2'-dihydroxy-5-aminobiphenyl; 2,3'-dihydroxy-5-aminobiphenyl; 2,4'-dihydroxy-5-aminobiphenyl; 2,5'-dihydroxy-5-aminobiphenyl; 2,6'-dihydroxy-5-aminobiphenyl; 2-hydroxy-5,2'-diaminobiphenyl; 2-hydroxy-5,3'-diaminobiphenyl; 2-hydroxy-5,4'-diaminobiphenyl; 2-hydroxy-5,5'-diaminobiphenyl; 2-hydroxy-5,6'-diaminobiphenyl; 2,2',3'-trihydroxy-5-aminobiphenyl; 2,2',4'-trihydroxy-5-aminobiphenyl; 2,2',5'-trihydroxy-5-aminobiphenyl; 2,2',6'-trihydroxy-5-aminobiphenyl; 2,3',4'-trihydroxy-5-aminobiphenyl; 2,3',5'-trihydroxy-5-aminobiphenyl; 2-hydroxy-5,2',3'-triaminobiphenyl; 2-hydroxy-5,2',4'-triaminobiphenyl; 2-hydroxy-5,2',5'-triaminobiphenyl; 2-hydroxy-5,2',6'-triaminobiphenyl; 2-hydroxy-5,3',4'-triaminobiphenyl; 2-hydroxy-5,3',5'-triaminobiphenyl; 2-hydroxy-5,3',4'-triaminobiphenyl; 2-hydroxy-5,3',5'-triaminobiphenyl; 2-hydroxy-5-amino-2'-aminomethylbiphenyl; 2-hydroxy-5-amino-2'-chlorobiphenyl; 2-hydroxy-5-amino-2'-cyanobiphenyl; 2-hydroxy-5-amino-2'-fluorobiphenyl; 2-hydroxy-5-amino-2'-methoxybiphenyl; 2-hydroxy-5-amino-2'-methylbiphenyl; 2-hydroxy-5-amino-2'-methylsulfanylbiphenyl; 2-hydroxy-5-amino-2'-nitrobiphenyl; 2-hydroxy-5-amino-3'-aminomethylbiphenyl; 2-hydroxy-5-amino-3'-chlorobiphenyl; 2-hydroxy-5-amino-3'-cyanobiphenyl; 2-hydroxy-5-amino-3'-fluorobiphenyl; 2-hydroxy-5-amino-3'-methoxybiphenyl; 2-hydroxy-5-amino-3'-methylbiphenyl, 2-hydroxy-5-amino-3'-methylsulfanylbiphenyl; 2-hydroxy-5-amino-3'-nitrobiphenyl; 2-hydroxy-5-amino-4'-aminomethylbiphenyl; 2-hydroxy-5-amino-4'-cyanobiphenyl; 2-hydroxy-5-amino-4'-fluorobiphenyl; 2-hydroxy-5-amino-4'-methoxybiphenyl; 2-hydroxy-5-amino-4'-methylbiphenyl; 2-hydroxy-5-amino-4'-methylsulfanylbiphenyl; 2-hydroxy-5-amino-4'-nitrobiphenyl; 2-hydroxy-5-amino-5'-aminomethylbiphenyl; 2-hydroxy-5-amino-5'-chlorobiphenyl; 2-hydroxy-5-amino-5'-cyanobiphenyl; 2-hydroxy-5-amino-5'-fluorobiphenyl; 2-hydroxy-5-amino-5'-methoxybiphenyl; 2-hydroxy-5-amino-5'-methylbiphenyl; 2-hydroxy-5-amino-5'-methylsulfanylbiphenyl; 2-hydroxy-5-amino-5'-nitrobiphenyl; 2-hydroxy-5-amino-6'-aminomethylbiphenyl; 2-hydroxy-5-amino-6'-chlorobiphenyl; 2-hydroxy-5-amino-6'-cyanobiphenyl; 2-hydroxy-5-amino-6'-fluorobiphenyl; 2-hydroxy-5-amino-6'-methoxybiphenyl; 2-hydroxy-5-amino-6'-methylbiphenyl; 2-hydroxy-5-amino-6'-methylsulfanylbiphenyl; 2-hydroxy-5-amino-6'-nitrobiphenyl; 2,2'-dihydroxy-5-amino-3'-methylbiphenyl; 2,2'-dihydroxy-5-amino-4'-methylbiphenyl; 2,2'-dihydroxy-5-amino-5'-methylbiphenyl; 2,2'-dihydroxy-5-amino-6'-methylbiphenyl; 2,3'-dihydroxy-5-amino-4'-methylbiphenyl; 2,3'-dihydroxy-5-amino-5'-methylbiphenyl; 2-hydroxy-5-amino-2',3'-dimethoxybiphenyl; 2-hydroxy-5-amino-2',3'-dimethylbiphenyl; 2-hydroxy-5-amino-2',4'-dimethoxybiphenyl; 2-hydroxy-5-amino-2',4'-dimethylbiphenyl; 2-hydroxy-5-amino-2',5'-dimethoxybiphenyl; 2-hydroxy-5-amino-2',5'-dimethylbiphenyl; 2-hydroxy-5-amino-2',6'-dimethoxybiphenyl; 2-hydroxy-5-amino-2'6'-dimethylbiphenyl; 2-hydroxy-5-amino-3',4'-dimethoxybiphenyl; 2-hydroxy-5-amino-3'4'-dimethylbiphenyl; 2-hydroxy-5-amino-3',5'-dimethoxybiphenyl; 2-hydroxy-5-amino-3',5'dimethylbiphenyl; 2-hydroxy-5-amino-2'-methoxy-3'-methylbiphenyl; 2-hydroxy-5-amino-2'-methoxy-4'-methylbiphenyl; 2-hydroxy-5-amino-2'-methoxy-5'-methylbiphenyl; 2-hydroxy-5-amino-2'-methoxy-6'-methylbiphenyl; 2-hydroxy-5-amino-3'-methoxy-4'-methylbiphenyl; 2-hydroxy-5-amino-3'-methoxy-5'-methylbiphenyl; 4-amino-2-benzo[1,3]dioxol-5-yl-phenol; 4-amino-2-benzo[2,4]dioxol-5-yl-phenol and 2-hydroxy-5-amino-4'-(2-hydroxyethoxy)biphenyl or physiologically compatible, water-soluble salts thereof.

6. Compound according to Claim 5, **characterized in that** it is chosen from 2,4'-dihydroxy-5-aminobiphenyl, 2-hydroxy-5-amino-4'-(2"-hydroxyethoxy)biphenyl, 2,4'-dihydroxy-5-amino-2'-methylbiphenyl, 2-hydroxy-5-amino-4'-(2"-hydroxyethyl)biphenyl, 2-hydroxy-5,4'-diaminobiphenyl or physiologically compatible salts thereof.

## Revendications

1. Agent pour la teinture par oxydation de fibres kératiniques, en particulier de cheveux humains, à base d'une combinaison substance de développement-substance de couplage **caractérisé en ce qu'**il contient, comme substance de développement, au moins un dérivé 2-hydroxy-5-aminobiphényle du groupe constitué par les dérivés 2-hydroxy-3-chloro-5-aminobiphényle ; 2-hydroxy-3-méthyl-5-aminobiphényle ; 2-hydroxy-5-aminobiphényle ; 2-hydroxy-4-chloro-5-aminobiphényle ; 2-hydroxy-4-méthyl-5-aminobiphényle ; 2,2'-dihydroxy-5-aminobiphényle ; 2,3'-dihydroxy-5-aminobiphényle ; 2,4'-dihydroxy-5-aminobiphényle ; 2,5'-dihydroxy-5-aminobiphényle ; 2,6'-dihydroxy-5-aminobiphényle ; 2-hydroxy-5,2'-diaminobiphényle ; 2-hydroxy-5,3'-diaminobiphényle ; 2-hydroxy-5,4'-diaminobiphényle ; 2-hydroxy-5,5'-diaminobiphényle ; 2-hydroxy-5,6'-diaminobiphényle ; 2,2',3'-trihydroxy-5-aminobiphényle ; 2,2',4'-trihydroxy-5-aminobiphényle ; 2,2',5'-trihydroxy-5-aminobiphényle ; 2,2',6'-trihydroxy-5-aminobiphényle ; 2,3',4'-trihydroxy-5-aminobiphényle ; 2,3',5'-trihydroxy-5-aminobiphényle ; 2-hydroxy-5,2',3'-triaminobiphényle ; 2-hydroxy-5,2',4'-triaminobiphényle ; 2-hydroxy-5,2',5'-triaminobiphényle ; 2-hydroxy-5,2',6'-triaminobiphényle ; 2-hydroxy-5,3',4'-triaminobiphényle ; 2-hydroxy-5,3',5'-triaminobiphényle ; 2-hydroxy-5,3',4'-triaminobiphényle ; 2-hydroxy-5,3',5'-triaminobiphényle ; 2-hydroxy-5-amino-2'-aminométhylbiphényle ; 2-hydroxy-5-amino-2'-chlorobiphényle ; 2-hydroxy-5-amino-2'-cyanobiphényle ; 2-hydroxy-5-amino-2'-fluorobiphényle ; 2-hydroxy-5-amino-2'-méthoxybiphényle ; 2-hydroxy-5-amino-2'-méthylbiphényle ; 2-hydroxy-5-amino-2'-méthylsulfanylbiphényle ; 2-hydroxy-5-amino-2'-nitrobiphényle ; 2-hydroxy-5-amino-3'-aminométhylbiphényle ; 2-hydroxy-5-amino-3'-chlorobiphényle ; 2-hydroxy-5-amino-3'-cyanobiphényle ; 2-hydroxy-5-amino-3'-fluorobiphényle ; 2-hydroxy-5-amino-3'-méthoxybiphényle ; 2-hydroxy-5-amino-3'-méthylbiphényle ; 2-hydroxy-5-amino-3'-méthylsulfanylbiphényle ; 2-hydroxy-5-amino-3'-nitrobiphényle ; 2-hydroxy-5-amino-4'-aminométhylbiphényle ; 2-hydroxy-5-amino-4'-chlorobiphényle ; 2-hydroxy-5-amino-4'-cyanobiphényle ; 2-hydroxy-5-amino-4'-fluorobiphényle ; 2-hydroxy-5-amino-4'-méthoxybiphényle ; 2-hydroxy-5-amino-4'-méthylbiphényle ; 2-hydroxy-5-amino-4'-méthylsulfanylbiphényle ; 2-hydroxy-5-amino-4'-nitrobiphényle, 2-hydroxy-5-amino-5'-aminométhylbiphényle ; 2-hydroxy-5-amino-5'-chlorobiphényle ; 2-hydroxy-5-amino-5'-cyanobiphényle ; 2-hydroxy-5-amino-5'-fluorobiphényle ; 2-hydroxy-5-amino-5'-méthoxybiphényle ; 2-hydroxy-5-amino-5'-méthylbiphényle ; 2-hydroxy-5-amino-5'-méthylsulfanylbiphényle ; 2-hydroxy-5-amino-5'-nitrobiphényle ; 2-hydroxy-5-amino-6'-aminométhylbiphényle ; 2-hydroxy-5-amino-6'-chlorobiphényle ; 2-hydroxy-5-amino-6'-cyanobiphényle ; 2-hydroxy-5-amino-6'-fluorobiphényle ; 2-hydroxy-5-amino-6'-méthoxybiphényle ; 2-hydroxy-5-amino-6'-méthylbiphényle ; 2-hydroxy-5-amino-6'-méthylsulfanylbiphényle ; 2-hydroxy-5-amino-6'-nitrobiphényle ; 2,2'-dihydroxy-5-amino-3'-méthylbiphényle ; 2,2'-dihydroxy-5-amino-4'-méthylbiphényle ; 2,2'-dihydroxy-5-amino-5'-méthylbiphényle 2,2'-dihydroxy-5-amino-6'-méthylbiphënyle ; 2,3'-dihydroxy-5-amino-4'-méthylbiphényle ; 2,3'-dihydroxy-5-amino-5'-méthylbiphényle ; 2-hydroxy-5-amino-2',3'-diméthoxybiphényle ; 2-hydroxy-5-amino-2',3'-diméthylbiphényle ; 2-hydroxy-5-amino-2',4'-diméthoxybiphényle ; 2-hydroxy-5-amino-2',4'-diméthylbiphényle ; 2-hydroxy-5-amino-2',5'-diméthoxybiphényle ; 2-hydroxy-5-amino-2',5'-diméthylbiphényle ; 2-hydroxy-5-amino-2',6'-diméthoxybiphényle ; 2-hydroxy-5-amino-2',6'-diméthylbiphényle ; 2-hydroxy-5-amino-3',4'-diméthoxybiphényle ; 2-hydroxy-5-amino-3',4' diméthylbiphényle ; 2-hydroxy-5-amino-3',5'-diméthoxybiphényle ; 2-hydroxy-5-amino-3',5'-diméthylbiphényle ; 2-hydroxy-5-amino-2'-méthoxy-3'-méthylbiphényle ; 2-hydroxy-5-amino-2'-méthoxy-4'-méthylbiphényle ; 2-hydroxy-5-amino-2'-méthoxy-5'-méthylbiphényle ; 2-hydroxy-5-amino-2'-méthoxy-6'-méthylbiphényle ; 2-hydroxy-5-amino-3'-méthoxy-4'-méthylbiphényle ; 2-hydroxy-5-amino-3'-méthoxy-5'-méthylbiphényle ; 4-amino-2-benzo[1,3]dioxol-5-ylphénol ; 4-amino-2-benzo[2,4]dioxol-5-ylphénol et 2-hydroxy-5-amino-4'-(2-hydroxyéthoxy)biphényle ou leurs sels solubles dans l'eau, physiologiquement acceptables.

2. Agent selon la revendication 1, **caractérisé en ce que** le dérivé 2-hydroxy-5-aminobiphényle est choisi parmi les dérivés 2-hydroxy-5-aminobiphényle ; 2,4'-dihydroxy-5-aminobiphényle, 2-hydroxy-5-amino-4'-(2"-hydroxyéthoxy)biphényle, 2,4'-dihydroxy-5-amino-2'-méthylbiphényle, 2-hydroxy-5-amino-4'-(2"-hydroxyéthyl)biphényle, 2-hydroxy-5,4'-diaminobiphényle ou leurs sels physiologiquement acceptables.

3. Agent selon la revendication 1 ou 2, **caractérisé en ce que** le dérivé 2-hydroxy-5-aminobiphényle est contenu en une quantité de 0,005 à 20,0% en poids.

4. Agent selon l'une quelconque des revendications 1 à 3, **caractérisé en ce qu'**il présente un pH de 6,5 à 11,5.

5. Dérivés 2-hydroxy-5-aminobiphényle qui sont choisis parmi les dérivés 2-hydroxy-3-chloro-5-aminobiphényle ; 2-hydroxy-3-méthyl-5-aminobiphényle ; 2-hydroxy-4-chloro-5-aminobiphényle ; 2-hydroxy-4-méthyl-5-aminobiphényle ; 2,2'-dihydroxy-5-aminobiphényle ; 2,3'-dihydroxy-5-aminobiphényle ; 2,4'-dihydroxy-5-aminobiphényle ; 2,5'-dihydroxy-5-aminobiphényle ; 2,6'-dihydroxy-5-aminobiphényle ; 2-hydroxy-5,2'-diaminobiphényle ; 2-hydroxy-5,3'-diaminobiphényle ; 2-hydroxy-5,4'-diaminobiphényle ; 2-hydroxy-5,5'-diaminobiphényle ; 2-hydroxy-5,6'-diaminobiphényle ; 2,2',3'-trihydroxy-5-aminobiphényle ; 2,2',4'-trihydroxy-5-aminobiphényle ; 2,2',5'-trihydroxy-5-aminobiphényle ; 2,2',6'-trihydroxy-5-aminobiphényle ; 2,3',4'-trihydroxy-5-aminobiphényle ; 2,3',5'-trihydroxy-5-aminobiphényle ; 2-hydroxy-5,2',3'-triaminobiphényle ; 2-hydroxy-5,2',4'-triaminobiphényle ; 2-hydroxy-5,2',5'-triaminobiphényle ; 2-hydroxy-5,2',6'-triaminobiphényle ; 2-hydroxy-5,3',4'-triaminobiphényle ; 2-hydroxy-5,3',5'-triaminobiphényle ; 2-hydroxy-5,3',4'-triaminobiphényle ; 2-hydroxy-5,3',5'-triaminobiphényle ; 2-hydroxy-5-amino-2'-aminométhylbiphényle ; 2-hydroxy-5-amino-2'-chlorobiphényle ; 2-hydroxy-5-amino-2'-cyanobiphényle ; 2-hydroxy-5-amino-2'-fluorobiphényle ; 2-hydroxy-5-amino-2'-méthoxybiphényle ; 2-hydroxy-5-amino-2'-méthylbiphényle ; 2-hydroxy-5-amino-2'-méthylsulfanylbiphényle ; 2-hydroxy-5-amino-2'-nitrobiphényle ; 2-hydroxy-5-amino-3'-aminométhylbiphényle ; 2-hydroxy-5-amino-3'-chlorobiphényle ; 2-hydroxy-5-amino-3'-cyanobiphényle ; 2-hydroxy-5-amino-3'-fluorobiphényle ; 2-hydroxy-5-amino-3'-méthoxybiphényle ; 2-hydroxy-5-amino-3'-méthylbiphényle ; 2-hydroxy-5-amino-3'-méthylsulfanylbiphényle ; 2-hydroxy-5-amino-3'-nitrobiphényle ; 2-hydroxy-5-amino-4'-aminométhylbiphényle ; 2-hydroxy-5-amino-4'-cyanobiphényle ; 2-hydroxy-5-amino-4'-fluorobiphényle ; 2-hydroxy-5-amino-4'-méthoxybiphényle ; 2-hydroxy-5-amino-4'-méthylbiphényle ; 2-hydroxy-5-amino-4'-méthylsulfanylbiphényle ; 2-hydroxy-5-amino-4'-nitrobiphényle, 2-hydroxy-5-amino-5'-aminométhylbiphényle ; 2-hydroxy-5-amino-5'-chlorobiphényle ; 2-hydroxy-5-amino-5'-cyanobiphényle ; 2-hydroxy-5-amino-5'-fluorobiphényle ; 2-hydroxy-5-amino-5'-méthoxybiphényle ; 2-hydroxy-5-amino-5'-méthylbiphényle ; 2-hydroxy-5-amino-5'-méthylsulfanylbiphényle ; 2-hydroxy-5-amino-5'-nitrobiphényle ; 2-hydroxy-5-amino-6'-aminométhylbiphényle ; 2-hydroxy-5-amino-6'-chlorobiphényle ; 2-hydroxy-5-amino-6'-cyanobiphényle ; 2-hydroxy-5-amino-6'-fluorobiphényle ; 2-hydroxy-5-amino-6'-méthoxybiphényle ; 2-hydroxy-5-amino-6'-méthylbiphényle ; 2-hydroxy-5-amino-6'-méthylsulfanylbiphényle ; 2-hydroxy-5-amino-6'-nitrobiphényle ; 2,2'-dihydroxy-5-amino-3'-méthylbiphényle ; 2,2'-dihydroxy-5-amino-4'-méthylbiphényle ; 2,2'-dihydroxy-5-amino-5'-méthylbiphényle ; 2,2'-dihydroxy-5-amino-6'-méthylbiphényle ; 2,3'-dihydroxy-5-amino-4'-méthylbiphényle ; 2,3'-dihydroxy-5-amino-5'-méthylbiphényle ; 2-hydroxy-5-amino-2',3'-diméthoxybiphényle ; 2-hydroxy-5-amino-2',3'-diméthylbiphényle ; 2-hydroxy-5-amino-2',4'-diméthoxybiphényle ; 2-hydroxy-5-amino-2',4'-diméthylbiphényle ; 2-hydroxy-5-amino-2',5'-diméthoxybiphényle ; 2-hydroxy-5-amino-2',5'-diméthylbiphényle ; 2-hydroxy-5-amino-2',6'-diméthoxybiphényle ; 2-hydroxy-5-amino-2',6'-diméthylbiphényle ; 2-hydroxy-5-amino-3',4'-diméthoxybiphényle ; 2-hydroxy-5-amino-3',4'-diméthylbiphényle ; 2-hydroxy-5-amino-3',5'-diméthoxybiphényle ; 2-hydroxy-5-amino-3',5'-diméthylbiphényle ; 2-hydroxy-5-amino-2'-méthoxy-3'-méthylbiphényle ; 2-hydroxy-5-amino-2'-méthoxy-4'-méthylbiphényle ; 2-hydroxy-5-amino-2'-méthoxy-5'-méthylbiphényle ; 2-hydroxy-5-amino-2'-méthoxy-6'-méthylbiphényle ; 2-hydroxy-5-amino-3'-méthoxy-4'-méthylbiphényle ; 2-hydroxy-5-amino-3'-méthoxy-5'-méthylbiphényle ; 4-amino-2-benzo[1,3]dioxol-5-ylphénol ; 4-amino-2-benzo[2,4]dioxol-5-ylphénol et 2-hydroxy-5-amino-4'-(2-hydroxyéthoxy)biphényle ou leurs sels solubles dans l'eau physiologiquement acceptables.

6. Composé selon la revendication 5, **caractérisé en ce qu'**il est choisi parmi les composés 2,4'-dihydroxy-5-aminobiphényle, 2-hydroxy-5-amino-4'-(2"-hydroxyéthoxy)biphényle, 2,4'-dihydroxy-5-amino-2'-méthylbiphényle, 2-hydroxy-5-amino-4'-(2"-hydroxyéthyl)biphényle, 2-hydroxy-5,4'-diaminobiphényle ou leurs sels physiologiquement acceptables.
